# EUROPEAN PATENT SPECIFICATION

(11) **EP 1 534 859 B1**
(45) Date of publication and mention of the grant of the patent: **24.11.2010**
(21) Application number: 03761972.3
(22) Date of filing: 20.06.2003
(51) Int. Cl.: C12Q 1/68, C12P 19/34, C07H 21/04

(54) **METHODS OF DETECTING SEQUENCE DIFFERENCES**
VERFAHREN ZUM NACHWEIS VON SEQUENZUNTERSCHIEDEN
METHODES DE DETECTION DE POLYMORPHISMES GENETIQUES

(30) Priority: 28.06.2002 US 392331 P
(43) Date of publication of application: 01.06.2005
(73) Proprietor: PrimeraDx, Inc., Mansfield, MA 02048 (US)
(72) Inventor: SLEPNEV, Vladimir, I., Cincinnati, OH 45244 (US)
(74) Representative: Dempster, Robert Charles
(86) International application number: PCT/US2003/019576
(87) International publication number: WO 2004/003136

(56) References cited:
- WO-A-01/29260
- WO-A-01/81631
- WO-A-03/002752
- US-A- 6 015 675
- PIGGEE C A ET AL: "Capillary electrophoresis for the detection of known point mutations by single-nucleotide primer extension and laser-induced fluorescence detection" JOURNAL OF CHROMATOGRAPHY A, ELSEVIER, AMSTERDAM, NL, vol. 781, no. 1-2, 26 September 1997 (1997-09-26), pages 367-375, XP004094567 ISSN: 0021-9673
- MCCLAY ET AL: 'High-throughput single-nucleotide polymorphism genotyping by fluorescent competitive allele-specific polymerase chain reaction (SNiPTag)' ANALYTICAL BIOCHEMISTRY vol. 301, 2002, pages 200 - 206, XP002973186
- MYAKISHEV ET AL: 'High-throughput SNP genotyping by allele-specific PCR with universal energy-transfer-labeled primers' GENOME RESEARCH vol. 11, 2001, pages 163 - 169, XP002242292

### SEQUENCE LISTING

<110> Slepnev, Vladimir I
<120> Methods of Detecting Sequence Differences
<130> 19781/2048
<140> Not yet assigned
   <141> 2003-06-20
<150> US 60/392,331
   <151> 2002-06-28
<160> 23
<170> PatentIn version 3.1
<210> 1
   <211> 33
   <212> DNA
   <213> Homo sapiens
<400> 1
   cgggctacta caacccttcg ctgacgccat aaa 33
<210> 2
   <211> 33
   <212> DNA
   <213> Homo sapiens
<400> 2
   tcaaactacg aacgcactca cagtcgcatc ata 33
<210> 3
   <211> 33
   <212> DNA
   <213> Homo sapiens
<400> 3
   ctcaggatac tcctcaatag ccatcgctgt agt 33
<210> 4
   <211> 46
   <212> DNA
   <213> Artificial
<220>
   <221> misc_feature
   <222> (1)..(46)
   <223> Synthetic primer sequence comprising human LHON-associated sequen ce linked to an artificial tag sequence.
<400> 4
   gttacaagat tctcacacgc taaggttcat agtagaagag cgatgg 46
<210> 5
   <211> 46
   <212> DNA
   <213> artificial
<220>
   <221> misc_feature
   <222> (1)..(46)
   <223> Synthetic primer sequence comprising human LHON-associated sequen ce linked to an artificial tag sequence.
<400> 5
   gttacaagat tctcacacgc taaggaaaaa gctattagtg ggagta 46
<210> 6
   <211> 46
   <212> DNA
   <213> artificial
<220>
   <221> misc_feature
   <222> (1)..(46)
   <223> Synthetic primer sequence comprising human LHON-associated sequen ce linked to an artificial tag sequence.
<400> 6
   gttacaagat tctcacacgc taaggtcggg tgtgttatta ttctga 46
<210> 7
   <211> 51
   <212> DNA
   <213> artificial
<220>
   <221> misc_feature
   <222> (1)..(51)
   <223> Synthetic primer sequence comprising human LHON-associated sequen ce linked to an artificial tag sequence.
<400> 7
   agttggcgaa gcagtcgcta gaagacgggc tactacaacc cttcgctgac g 51
<210> 8
   <211> 51
   <212> DNA
   <213> artificial
<220>
   <221> misc_feature
   <222> (1)..(51)
   <223> Synthetic primer sequence comprising human LHON-associated sequen ce linked to an artificial tag sequence.
<400> 8
   gatgctggtg tggctggtgt tcccgcgggc tactacaacc cttcgctgac a 51
<210> 9
   <211> 52
   <212> DNA
   <213> artificial
<220>
   <221> misc_feature
   <222> (1)..(52)
   <223> Synthetic primer sequence comprising human LHON-associated sequen ce linked to an artificial tag sequence.
<400> 9
   ggttggttgc acactggaga tattggcggg ctactacaac ccttcgctga ct 52
<210> 10
   <211> 51
   <212> DNA
   <213> artificial
<220>
   <221> misc_feature
   <222> (1)..(51)
   <223> Synthetic primer sequence comprising human LHON-associated sequen ce linked to an artificial tag sequence.
<400> 10
   ctggagcatc tggaaaagta gtacccgggc tactacaacc cttcgctgac c 51
<210> 11
   <211> 51
   <212> DNA
   <213> artificial
<220>
   <221> misc_feature
   <222> (1)..(51)
   <223> Synthetic primer sequence comprising human LHON-associated sequen ce linked to an artificial tag sequence.
<400> 11
   agttggcgaa gcagtcgcta gaagatcaaa ctacgaacgc actcacagtc g 51
<210> 12
   <211> 51
   <212> DNA
   <213> artificial
<220>
   <221> misc_feature
   <222> (1)..(51)
   <223> Synthetic primer sequence comprising human LHON-associated sequen ce linked to an artificial tag sequence.
<400> 12
   gatgctggtg tggctggtgt tcccgtcaaa ctacgaacgc actcacagtc a 51
<210> 13
   <211> 52
   <212> DNA
   <213> artificial
<220>
   <221> misc_feature
   <222> (1)..(52)
   <223> Synthetic primer sequence comprising human LHON-associated sequen ce linked to an artificial tag sequence.
<400> 13
   ggttggttgc acactggaga tattggtcaa actacgaacg cactcacagt ct 52
<210> 14
   <211> 51
   <212> DNA
   <213> artificial
<220>
   <221> misc_feature
   <222> (1)..(51)
   <223> Synthetic primer sequence comprising human LHON-associated sequen ce linked to an artificial tag sequence.
<400> 14
   ctggagcatc tggaaaagta gtacctcaaa ctacgaacgc actcacagtc c 51
<210> 15
   <211> 51
   <212> DNA
   <213> artificial
<220>
   <221> misc feature
   <222> (1)..(51)
   <223> Synthetic primer sequence comprising human LHON-associated sequen ce linked to an artificial tag sequence.
<400> 15
   agttggcgaa gcagtcgcta gaagactcag gatactcctc aatagccatc g 51
<210> 16
   <211> 51
   <212> DNA
   <213> artificial
<220>
   <221> misc_feature
   <222> (1)..(51)
   <223> Synthetic primer sequence comprising human LHON-associated sequen ce linked to an artificial tag sequence.
<400> 16
   gatgctggtg tggctggtgt tcccgctcag gatactcctc aatagccatc a 51
<210> 17
   <211> 52
   <212> DNA
   <213> artificial
<220>
   <221> misc_feature
   <222> (1)..(52)
   <223> Synthetic primer sequence comprising human LHON-associated sequen ce linked to an artificial tag sequence.
<400> 17
   ggttggttgc acactggaga tattggctca ggatactcct caatagccat ct 52
<210> 18
   <211> 51
   <212> DNA
   <213> artificial
<220>
   <221> misc_feature
   <222> (1)..(51)
   <223> Synthetic primer sequence comprising human LHON-associated sequen ce linked to an artificial tag sequence.
<400> 18
   ctggagcatc tggaaaagta gtaccctcag gatactcctc aatagccatc c 51
<210> 19
   <211> 25
   <212> DNA
   <213> artificial
<220>
   <221> misc_feature
   <222> (1)..(25)
   <223> Synthetic amplification primer that hybridizes to artificial tag sequence incorporated by primer extension primers.
<400> 19
   gttacaagat tctcacacgc taagg 25
<210> 20
   <211> 25
   <212> DNA
   <213> artificial
<220>
   <221> misc_feature
   <222> (1)..(25)
   <223> Synthetic amplification primer that hybridizes to artificial tag sequence incorporated by primer extension primers.
<400> 20
   agttggcgaa gcagtcgcta gaaga 25
<210> 21
   <211> 25
   <212> DNA
   <213> artificial
<220>
   <221> misc_feature
   <222> (1)..(25)
   <223> Synthetic amplification primer that hybridizes to artificial tag sequence incorporated by primer extension primers.
<400> 21
   gatgctggtg tggctggtgt tcccg 25
<210> 22
   <211> 26
   <212> DNA
   <213> artificial
<220>
   <221> misc_feature
   <222> (1)..(26)
   <223> Synthetic amplification primer that hybridizes to artificial tag sequence incorporated by primer extension primers.
<400> 22
   ggttggttgc acactggaga tattgg 26
<210> 23
   <211> 25
   <212> DNA
   <213> artificial
<220>
   <221> misc_feature
   <222> (1)..(25)
   <223> Synthetic amplification primer that hybridizes to artificial tag sequence incorporated by primer extension primers.
<400> 23
   ctggagcatc tggaaaagta gtacc 25

## Description

### FIELD OF THE INVENTION

The invention relates to molecular genetic methods for the identification of sequence differences in the genome of an individual relative to the sequences of a population of individuals. More particularly, the invention relates to methods for the identification of single nucleotide differences in genomic sequences.

### BACKGROUND OF THE INVENTION

The nucleic acids comprising the genome of an organism contain the genetic information for that organism. Variability in gene sequences between individuals accounts for many of the obvious phenotypic differences (such as pigmentation of hair, skin, etc.) and many non-obvious ones (such as drug tolerance and disease susceptibility). Even minute changes in a nucleotide sequence, including single base pair substitutions, can have a significant effect on the quality or quantity of a protein. Single nucleotide changes are referred to as single nucleotide polymorphisms or simply SNPs, and the site at which the SNP occurs is referred to herein as a polymorphic site. DNA polymorphisms are located throughout the genome, within and between genes, and the various forms may or may not result in differential gene function (as determined by comparing the function of two alternative forms of the same sequence). Most polymorphisms do not alter gene function and are termed "neutral" polymorphisms. Others do have affect gene function, for example, by changing the amino acid sequence of a protein, or by altering control sequences such as promoters or RNA splicing or degradation signals, and are more commonly referred to as mutations. Diseases associated with SNPs include: sickle cell anemia, β-thalassemias, diabetes, cystic fibrosis, hyperlipoproteinemia, a wide variety of autoimmune diseases, and the formation of some oncogenes, e.g., mutant p53. In addition to causing or affecting disease states, point mutations can cause altered pathogenicity or susceptibility to disease and resistance to therapeutics.

The ability to detect specific nucleotide alterations or mutations in DNA sequences is useful for a number of medical and non-medical purposes. Methods capable of identifying nucleotide alterations permit screening and diagnosis of diseases associated with SNPs. Polymorphisms are also useful in genetic studies to identify genes involved with a disease. If a polymorphism alters the function of one or more genes such that disease susceptibility is increased, the polymorphism will be present more often in individuals with the disease relative to those without the disease. Statistical methods can be used to evaluate polymorphism frequencies found in diseased relative to normal populations, and can facilitate the establishment of a causal link between a polymorphism and a disease phenotype.

Methods that can quickly identify sequence variations that correlate with disease are also valuable in permitting prophylactic measures, in the assessment of the likelihood of developing disease and in evaluating the prognosis of such disease. Non-medical applications of SNPs include, for example, the detection of microorganisms or particular strains of them, and in forensic analysis.

Central to the usefulness of SNPs is the ability to determine the genotype of an individual with respect to known SNPs. A number of approaches to the problem have been taken. For example, some polymorphisms fortuitously result in changes in restriction endonuclease cleavage sites, thereby changing the pattern of fragments observed when a digested genomic DNA sample is separated by electrophoresis. This is the basis for Restriction Fragment Length Polymorphism analysis, or RFLP analysis. RFLP analysis is limited in that it can only detect those changes that affect a restriction endonuclease cleavage site, and the method is dependent upon gel electrophoresis and staining, which limits throughput.

Single-strand conformational polymorphism (SSCP) analysis can also detect SNPs in an amplified DNA fragment. In this method, the amplified fragment is denatured then allowed to re-anneal during electrophoresis in non-denaturing polyacrylamide gels. The presence of single nucleotide sequence changes can cause a detectable change in the conformation and electrophoretic migration of a sample relative to wild-type sequence. This method is limited in its dependence upon polyacrylamide gel electrophoresis.

Hybridization-based methods employ allele-specific oligonucleotide (ASO) probes (see, e.g., European Patent Publications EP-237362 and EP-32931 1). The hybridization-based methods include, for example, detection based on ribonuclease A cleavage at mismatches in probe RNA:sample DNA duplexes or denaturing gradient gel electrophoresis for mismatches in probe DNA:sample DNA duplexes (reviewed in Landegren et al., Science 242:229-237, 1988; Rossiter et al, J. Biol. Chem. 265:12753-12756, 1990).

Other methods of genotyping SNPs employ allele-specific amplification (see, e.g., U.S. Pat. Nos. 5,521,301; 5,639,611; and 5,981,176), mini-sequencing methods, quantitative RT-PCR methods (eg., the so-called "TaqMan assays"; see, e.g., U.S. Pat. No. 5,210,015 to Gelfand, U.S. Pat. No. 5,538,848 to Livak, et al., and U.S. Pat. No. 5,863,736 to Haaland, as well as Heid, C.A., et al. Genome Research, 6:986-994 (1996); Gibson, U.E. M, et al., Genome Research 6:995-1001 (1996); Holland, P. M., et al. Proc. Natl. Acad. Sci. USA 88:7276-7280, (1991); and Livak, K. J., et al., PCR Methods and Applications 357-362 (1995)), and single nucleotide primer extension (SNuPE) assays (e.g., U.S. Pat No. 5,846,710) and related extension assays (e.g., U.S. Pat. Nos. 6,004,744; 5,888,819; 5,856,092; 5,710,028 and 6,013,431). There is a need in the art for improved SNP genotyping assays.

Most SNP genotyping methods rely at some point upon PCR amplification, either to generate enough material for analysis (e.g., SSCP analysis) or to differentially amplify one form over another so as to detect differences (e.g., the primer extension assays). In order to increase the throughput of PCR-based methods, efforts are being focused on multiplexing the reactions so that multiple SNPs can be detected in a single set of reactions. Multiplexing by simply adding primer pairs specific for multiple SNP-containing fragments faces problems caused by primer interactions that lead to inefficient amplification of target fragments and to the generation of artifact fragments. There is a need in the art for improved multiplex SNP genotyping methods.

Capillary electrophoresis (CE) has been used to examine SNPs. One study used CE to analyze the results of a single nucleotide polymerase extension assay (Piggee et al., 1997, J. Chromatography A. 781: 367-375). In that study, PCR-amplified DNA containing a known SNP was analyzed by hybridization of a primer immediately adjacent to the polymorphic site and extension of the primer with a single fluorescently labeled chain terminator, followed by CE separation and detection of the incorporated label. In another study, PCR-amplified DNA containing a known SNP was extended with one of two identically fluorescently labeled chain terminators, followed by CE separation and detection of incorporated label. The identities of incorporated terminators are determined based on sequence-specific differences in CE migration for oligonucleotides. McClay et al. (2002, Anal. Biochem. 301: 200-206) describe an SNP genotyping assay involving PCR using a set of two differentially fluorescently labeled primers differing in their 3'-terminal base with a common upstream primer, followed by CE and fluorescent detection. Throughput was increased by mixing amplification products of different sizes and electrophoresing together.

U.S. Patent No. 6,074,831 teaches the use of CE for the concurrent separation of molecules partitioned into subsets according to graph theory techniques, and the application of the method to SNP genotyping.

U.S. Patent No. 6,322,980 describes the use of CE in an SNP detection method using the exonuclease activity of a polymerase to release a fluorescent label from a primer hybridized to the polymorphic site. U.S. Patent No. 6,270,973 also describes the use of CE separation in an SNP genotyping method involving nucleic acid probe depolymerizing activity.

U.S. Patent No. 6,312,893 describes a sequencing method that generates organically tagged fragments in which the tag correlates with a particular nucleotide. Fragments are separated by CE, followed by tag cleavage from the fragments and detection of cleaved tags by non-fluorescent spectrometry or potentiometry.

U.S. Patent No. 6,156,178 describes the use of CE in an SNP detection method using a depolymerizing activity to release an identifier nucleotide from a primer hybridized to the polymorphic site.

None of the above methods uses nucleic acid sequence tags in either primer extension or amplification steps, different primers for extension and amplification, common amplification primer sets or real-time amplification monitoring and detection.

### SUMMARY OF THE INVENTION

The present invention provides methods and kits as defined in the appended claims: The invention provides methods useful for genotyping nucleic acid samples with regard to sequence differences. In a preferred aspect, the methods are useful for the determination of single nucleotide differences, e.g., single nucleotide polymorphisms. The methods of the invention use PCR amplification of primer extension products comprising heterologous sequence tags, followed by capillary electrophoretic size separation and detection of the amplified extension products. In one aspect, the size separations and product detection are performed in real time. Because the CE separation and detection techniques provide information inluding the amplified fragment size and the identity of label present on any given amplification product, the disclosed methods are particularly well suited for simultaneously analyzing samples for genotype with regard to multiple known SNPs. Each known SNP can be detected by the amplification of a discretely sized amplification fragment bearing a distinguishably labeled sequence tag that specifically correlates with the presence of a particular nucleotide at that polymorphic site. Methods according to the invention also have the advantage of requiring one set of amplification primers for the detection of multiple SNPs, thereby reducing the impact of problems related to the use of multiple different amplification primers.

Described herein is a method of determining for a given nucleic acid sample, the identity of the nucleotide at a known polymorphic site, the method comprising: a) subjecting to an amplification regimen a population of primer extension products generated from a nucleic acid sample, each primer extension product comprising a tag sequence, which tag sequence specifically corresponds to the presence of one specific nucleotide at a known polymorphic site, wherein the amplification regimen is performed using an upstream amplification primer and a set of distinguishably labeled downstream amplification primers, each member of the set of downstream amplification primers comprising a tag sequence comprised by a member of the population of primer extension products and a distinguishable label, wherein each distinguishable label specifically corresponds to the presence of a specific nucleotide at the polymorphic site; and b) detecting incorporation of a distinguishable label into a nucleic acid molecule, thereby to determine the identity of the nucleotide at the polymorphic site.

In one embodiment, the distinguishable label is a fluorescent label.

In another embodiment step (b) comprises separating nucleic acid molecules made during the amplification regimen by size and/or by charge. In a preferred embodiment the separating comprises capillary electrophoresis.

In another embodiment the amplification regimen comprises at least two amplification reaction cycles, wherein each cycle comprises the steps of: 1) nucleic acid strand separation; 2) oligonucleotide primer annealing; and 3) polymerase extension of annealed primers. In a preferred embodiment the method further comprises the steps, during the amplification regimen and after at least one of the reaction cycles, of removing an aliquot of the amplification reaction, separating nucleic acid molecules by size and/or by charge, and detecting the incorporation of a distinguishable label, wherein the detecting determines the identity of the nucleotide at the polymorphic site. In a further preferred embodiment the removing, separating and detecting are performed after each cycle in the regimen. In a further preferred embodiment the separating comprises capillary electrophoresis.

In another embodiment, steps (a) and (b) are performed in a modular apparatus comprising a thermal cycler, a sampling device, a capillary electrophoresis device and a fluorescence detector.

In another embodiment the tag sequence comprises 15 to 40 nucleotides.

In another embodiment the set of distinguishably labeled downstream amplification primers consists of: a primer that comprises a tag sequence that specifically corresponds to the presence of A at the polymorphic site; a primer that comprises a tag sequence that specifically corresponds to the presence of C at the polymorphic site; a primer that comprises a tag sequence that specifically corresponds to the presence of G at the polymorphic site; and a primer that comprises a tag sequence that specifically corresponds to the presence of T at the polymorphic site.

In another embodiment the set of distinguishably labeled downstream amplification primers consists of a pair of oligonucleotides, one comprising a tag sequence that specifically corresponds to a first allele of the polymorphic site and one comprising a tag sequence that specifically corresponds to a second allele of the polymorphic site.

Another embodiment further comprises the step, before step (a), of removing primers not incorporated when the population of primer extension products was made. In a further preferred embodiment the step of removing primers comprises degrading the primers not incorporated when the population of primer extension products was made. In a further preferred embodiment the degrading is performed using a heat labile exonuclease. In a further preferred embodiment the heat labile exonuclease is selected from the group consisting of Exonuclease I and Exonuclease VII. In a further preferred embodiment wherein the heat labile exonuclease is thermally inactivated before continuing to step (a).

Described herein is a method of determining, for a given nucleic acid sample, the identities of the nucleotides at a set of known polymorphic sites to be interrogated, the method comprising: a) subjecting to an amplification regimen, a population of primer extension products generated from a nucleic acid sample, each primer extension product comprising a member of a set of tag sequences, which tag sequence specifically corresponds to the presence of one specific nucleotide at a known polymorphic site, wherein the amplification regimen is performed using one upstream amplification primer for each sequence comprising a known polymorphic site to be interrogated, and a set of distinguishably labeled downstream amplification primers, each member of the set of downstream amplification primers comprising a tag sequence comprised by a member of the population of primer extension products and a distinguishable label that specifically corresponds to the presence of a specific nucleotide at the polymorphic site, and wherein the upstream amplification primers are selected such that each polymorphic site of the set of known polymorphic sites to be interrogated corresponds to a distinctly sized amplification product; and b) detecting incorporation of a distinguishable label in distinctly sized amplification products, thereby to determine the identity of the nucleotide at each polymorphic site.

In one embodiment, the distinguishable label is a fluorescent label.

In another embodiment step (b) comprises separating nucleic acid molecules made during the amplification regimen by size and/or by charge. In a preferred embodiment the separating comprises capillary electrophoresis.

In one embodiment the amplification regimen comprises at least two amplification reaction cycles, wherein each cycle comprises the steps of: 1) nucleic acid strand separation; 2) oligonucleotide primer annealing; and 3) polymerase extension of annealed primers.

A preferred embodiment further comprises the steps, during the amplification regimen and after at least one of the reaction cycles, of removing an aliquot of the amplification reaction, separating nucleic acid molecules by size and/or by charge, and detecting the incorporation of a the distinguishable label, wherein the detecting determines the identity of the nucleotide at the polymorphic site. In a further preferred embodiment the removing, separating and detecting are performed after each cycle in the regimen. In a further preferred embodiment the separating comprises capillary electrophoresis.

In another embodiment steps (a) and (b) are performed in a modular apparatus comprising a thermal cycler, a sampling device, a capillary electrophoresis device and a fluorescent detector.

In another embodiment the tag sequence comprises 15 to 40 nucleotides.

In another embodiment the set of distinguishably labeled downstream amplification primers consists of: a subset that comprises a tag sequence that specifically corresponds to the presence of A at the polymorphic site; a subset that comprises a tag sequence that specifically corresponds to the presence of C at the polymorphic site; a subset that comprises a tag sequence that specifically corresponds to the presence of G at the polymorphic site; and a subset that comprises a tag sequence that specifically corresponds to the presence of T at the polymorphic site.

Another embodiment further comprises the step, before step (a), of removing primers not incorporated when the population of primer extension products was made. In a preferred embodiment the step of removing primers comprises degrading the primers not incorporated when the population of primer extension products was made. In a further preferred embodiment the degrading is performed using a heat labile exonuclease. In a further preferred embodiment the heat labile exonuclease is selected from the group consisting of Exonuclease I and Exonuclease VII. In a further preferred embodiment the heat labile exonuclease is thermally inactivated before continuing to step (a).

Described herein is a method of determining, for a given nucleic acid sample, the identities of the nucleotides at a set of known polymorphic sites to be interrogated, the method comprising: a) subjecting to an amplification regimen, a population of primer extension products generated from a nucleic acid sample, each primer extension product comprising a first tag sequence or its complement and a member of a set of second tag sequences or its complement, the presence of which second tag sequence or its complement specifically corresponds to the presence of one specific nucleotide at a known polymorphic site, wherein for each polymorphic site in the set of polymorphic sites, the first tag sequence is located at a distinct distance 5' of the polymorphic site, relative to the distance of the first tag sequence from a polymorphic site on molecules in the sample containing other polymorphic sites, wherein the amplification regimen is performed using an upstream amplification primer comprising the first tag sequence, and a set of distinguishably labeled downstream amplification primers, each member of the set of downstream amplification primers comprising a tag sequence comprised by a member of the population of primer extension products and a distinguishable label that specifically corresponds to the presence of a specific nucleotide at the polymorphic site, and wherein the upstream amplification primers are selected such that each polymorphic site of the set of known polymorphic sites to be interrogated corresponds to a distinctly sized amplification product; and b) detecting incorporation of a distinguishable label in distinctly sized amplification products, thereby to determine the identity of the nucleotide at each the polymorphic site.

In one embodiment, the distinguishable label is a fluorescent label.

In another embodiment step (b) comprises separating nucleic acid molecules made during the amplification regimen by size and/or by charge. In a preferred embodiment wherein the separating comprises capillary electrophoresis.

In another embodiment the amplification regimen comprising at least two amplification reaction cycles, wherein each cycle comprises the steps of: 1) nucleic acid strand separation; 2) oligonucleotide primer annealing; and 3) polymerase extension of annealed primers. A preferred embodiment further comprises the steps, during the amplification regimen and after at least one of the reaction cycles, of removing an aliquot of the amplification reaction, separating nucleic acid molecules by size and/or by charge, and detecting the incorporation of a distinguishable label, wherein the detecting determines the identity of the nucleotide at the polymorphic site. In a further preferred embodiment the removing, separating and detecting are performed after each cycle in the regimen. In a further preferred embodiment the separating comprises capillary electrophoresis.

In another embodiment steps (a) and (b) are performed in a modular apparatus comprising a thermal cycler, a sampling device, a capillary electrophoresis device and a fluorescent detector.

In another embodiment the tag sequence comprises 15 to 40 nucleotides.

In another embodiment the set of distinguishably labeled downstream amplification primers consists of: a subset that comprises a tag sequence that specifically corresponds to the presence of A at the polymorphic site; a subset that comprises a tag sequence that specifically corresponds to the presence of C at the polymorphic site; a subset that comprises a tag sequence that specifically corresponds to the presence of G at the polymorphic site; and a subset that comprises a tag sequence that specifically corresponds to the presence of T at the polymorphic site.

Another embodiment further comprises the step, before step (a), of removing primers not incorporated when the population of primer extension products was made. In a preferred embodiment the step of removing primers comprises degrading the primers not incorporated when the population of primer extension products was made. In a further preferred embodiment, the degrading is performed using a heat labile exonuclease. In a further preferred embodiment the heat labile exonuclease is selected from the group consisting of Exonuclease I and Exonuclease VII. In a further preferred embodiment the heat labile exonuclease is thermally inactivated before continuing to step (a).

Described herein is a method of determining the identify of a single nucleotide at a known polymorphic site, the method comprising: I) providing a nucleic acid sample comprising the polymorphic site; II) separating the strands of the nucleic acid sample and re-annealing in the presence of: a) a first oligonucleotide primer comprising a 3' region that hybridizes to a sequence at a known distance upstream of the known polymorphic site, the first oligonucleotide primer comprising a first sequence tag located 5' of the 3' region; and b) a set of second oligonucleotide primers, wherein each member of the set comprises: i) a region that hybridizes 3' of and adjacent to the polymorphic site; ii) a variable 3' terminal nucleotide, wherein, when the member is hybridized to the known sequence, the 3' terminal nucleotide is opposite the polymorphic site, and wherein, if and only if the 3' terminal nucleotide is complementary to the nucleotide at the polymorphic site, the 3' terminal nucleotide base pairs with the nucleotide at the polymorphic site; and iii) a tag sequence that corresponds to the varaible 3'-terminal nucleotide of (ii), the tag sequence located 5' of the region of (i) on the member; III) contacting the annealed oligonucleotides resulting from step (II) with a nucleic acid polymerase under conditions that permit the extension of an annealed oligonucleotide such that extension products are generated, wherein the primer extension product from the first oligonucleotide primer, when separated from its complement, can serve as a template for the synthesis of the extension product of a member of the set of second oligonucleotide primers, and vice versa; IV) repeating strand separating and contacting steps (II) and (III) two times, such that a population of nucleic acid molecules is generated that comprises both a sequence identical to or complementary to the first oligonucleotide and a sequence identical to or complementary to one of the members of the second set of oligonucleotides; V) contacting the population generated in step (IV) with a heat-labile exonuclease under conditions permitting the degradation of non-annealed oligonucleotide primers, such that the primers are degraded; VI) thermally inactivating the heat-labile exonuclease; VII) subjecting the population of nucleic acid molecules to an amplification regimen, wherein the amplification regimen is performed using an upstream amplification primer comprising the first sequence tag comprised by the first oligonucleotide primer, and a set of downstream amplification primers, each member of the set of downstream amplification primers comprising a tag comprised by a member of the set of second oligonucleotide primers and a distinguishable label; and VIII) detecting incorporation of at least one distinguishable label, thereby determining the identity of the nucleotide at the known polymorphic site.

In one embodiment, the distinguishable label is a fluorescent label.

In another embodiment step (VIII) comprises separating nucleic acid molecules made during the amplification regimen by size and/or by charge. In a preferred embodiment the separating comprises capillary electrophoresis.

In another embodiment the amplification regimen comprises at least two amplification reaction cycles, wherein each cycle comprises the steps of: 1) nucleic acid strand separation; 2) oligonucleotide primer annealing; and 3) polymerase extension of annealed primers. A preferred embodiment further comprises the steps, during the amplification regimen and after at least one of the reaction cycles, of removing an aliquot of the amplification reaction, separating nucleic acid molecules by size and/or by charge, and detecting the incorporation of a distinguishable label, wherein the detecting determines the identity of the nucleotide at the polymorphic site. In another preferred embodiment the removing, separating and detecting are performed after each cycle in the regimen.

In another embodiment steps I-VIII are performed in a modular apparatus comprising a thermal cycler, a sampling device, a capillary electrophoresis device and a fluorescent detector.

In another embodiment the tag sequences each comprise 15 to 40 nucleotides.

In another embodiment the 3' region that hybridizes to a sequence at a known distance upstream of the known polymorphic site comprises 10-30 nucleotides.

In another embodiment the region that hybridizes 3' of and adjacent to the polymorphic site comprises 10-30 nucleotides.

In another embodiment the set of downstream amplification primers consists of: a subset that comprises a tag sequence that specifically corresponds to the presence of A at the polymorphic site; a subset that comprises a tag sequence that specifically corresponds to the presence of C at the polymorphic site; a subset that comprises a tag sequence that specifically corresponds to the presence of G at the polymorphic site; and a subset that comprises a tag sequence that specifically corresponds to the presence of T at the polymorphic site.

Described herein is a method of determining the identities of single nucleotides present at a group of known polymorphic sites, the method comprising: I) providing a nucleic acid sample comprising the group of polymorphic sites; II) separating the strands of the nucleic acid sample and re-annealing in the presence of: a) a set of first oligonucleotide primers each comprising a 3' region that hybridizes to a sequence at a known distance upstream of a known polymorphic site, each member of the set of first oligonucleotide primers comprising a common sequence tag located 5' of the 3' region, and each member of the set of first oligonucleotide primers selected such that a distinctly sized amplification product is generated for each polymorphic site in the group of known polymorphic sites; and b) a set of downstream amplification primers comprising, in 5' to 3' order: i) a sequence tag selected from the group consisting of a tag specifically corresponding to G as the 3'-terminal nucleotide of the primer; a tag specifically corresponding to A as the 3'-terminal nucleotide of the primer; a tag specifically corresponding to T as the 3'-terminal nucleotide of the primer; and a tag specifically corresponding to C as the 3'-terminal nucleotide of the primer; ii) a region that specifically hybridizes to a sequence adjacent to and 3' of a polymorphic site in the group of polymorphic sites, wherein the set of downstream amplification primers comprises a subset of primers comprising a region that specifically hybridizes adjacent to the polymorphic site for each polymorphic site in the group of polymorphic sites; and iii) a 3' terminal nucleotide selected from G, A, T or C, wherein the terminal nucleotide specifically corresponds to the sequence tag described in (i) on that downstream amplification primer, and wherein when the downstream amplification primer is hybridized to the sequence adjacent to and 3' of a polymorphic site, the 3' terminal nucleotide is opposite the polymorphic site; III) contacting the annealed oligonucleotides resulting from step (II) with a nucleic acid polymerase under conditions that permit the extension of an annealed oligonucleotide such that extension products are generated, wherein the primer extension product from the first oligonucleotide primer, when separated from its complement, can serve as a template for the synthesis of the extension product of as member of the set of second oligonucleotide primers, and vice versa; IV) repeating strand separating and contacting steps (II) and (III) two times, such that a reaction mixture comprising a population of nucleic acid molecules is generated that comprises both a sequence identical to or complementary to the first oligonucleotide and a sequence identical to or complementary to a member of the set of downstream amplification primers; V) contacting the population gnerated in step (IV) with a heat-labile exonuclease under conditions permitting the degradation of non-annealed oligonucleotide primers, such that non-annealed primers are degraded; VI) thermally inactivating the heat-labile exonuclease; VII) subjecting the population of nucleic acid molecules to an amplification regimen, wherein the amplification regimen is performed using an upstream amplification primer comprising the common sequence tag comprised by the first oligonucleotide primer, and a set of downstream amplification primers, each member of the set of downstream amplification primers comprising a tag comprised by a member of the set of second oligonucleotide primers and a distinguishable label; and VIII) detecting incorporation of at least one distinguishable label, thereby determining the identities of the nucleotides present at the known polymorphic sites.

In one embodiment the distinguishable label is a fluorescent label.

In one embodiment the step (VIII) comprises separating nucleic acid molecules made during the amplification regimen by size and/or by charge. In a preferred embodiment the separating comprises capillary electrophoresis.

In another embodiment the amplification regimen comprising at least two amplification reaction cycles, wherein each cycle comprises the steps of: 1) nucleic acid strand separation; 2) oligonucleotide primer annealing; and 3) polymerase extension of annealed primers. A preferred embodiment further comprises the steps, during the amplification regimen and after at least one of the reaction cycles, of removing an aliquot of the amplification reaction, separating nucleic acid molecules by size and/or by charge, and detecting the incorporation of a distinguishable label, wherein the detecting determines the identity of the nucleotide at the polymorphic site. In a further preferred embodiment the removing, separating and detecting are performed after each cycle in the regimen.

In another embodiment steps I-VIII are performed in a modular apparatus comprising a thermal cycler, a sampling device, a capillary electrophoresis device and a fluorescent detector.

In another embodiment the tag sequences each comprise 15 to 40 nucleotides.

In another embodiment the 3' region that hybridizes to a sequence at a known distance upstream of the known polymorphic site comprises 10-30 nucleotides.

In another embodiment the region that hybridizes 3' of and adjacent to the polymorphic site comprises 10-30 nucleotides.

In another embodiment the set of distinguishably labeled downstream amplification primers consists of: a subset that comprises a tag sequence that specifically corresponds to the presence of A at the polymorphic site; a subset that comprises a tag sequence that specifically corresponds to the presence of C at the polymorphic site; a subset that comprises a tag sequence that specifically corresponds to the presence of G at the polymorphic site; and a subset that comprises a tag sequence that specifically corresponds to the presence of T at the polymorphic site.

The invention further encompasses a kit for the determination of the nucleotide present at a polymorphic site present on a nucleic acid sample, the kit comprising a set of upstream primers comprising: a) a first primer comprising a 5'-tag sequence and 3' sequence sufficient to specifically hybridize at a known distance upstream of a known polymorphic site; and b) a set of 4 downstream second primers, comprising in 5' to 3' order: i) a sequence tag selected from the group consisting of a tag specifically corresponding to G as the 3'-terminal nucleotide of the primer; a tag specifically corresponding to A as the 3'-terminal nucleotide of the primer; a tag specifically corresponding to T as the 3'-terminal nucleotide of the primer; and a tag specifically corresponding to C as the 3'-terminal nucleotide of the primer; ii) a region that specifically hybridizes to a sequence adjacent to and 3' of a polymorphic site in the group of polymorphic sites, wherein the set of downstream amplification primers comprises a subset of primers comprising a region that specifically hybridizes adjacent to the polymorphic site for each polymorphic site in the group of polymorphic sites; and iii) a 3' terminal nucleotide selected from G, A, T or C, wherein the terminal nucleotide specifically corresponds to the sequence tag described in (i) on that downstream amplification primer, and wherein when the downstream amplification primer is hybridized to the sequence adjacent to and 3' of a polymorphic site, the 3' terminal nucleotide is opposite the polymorphic site.

One embodiment further comprises a set of 5 primers lacking sequence specific for a gene in the genome of the organism being examined for polymorphisms, the primers comprising a primer comprising the tag sequence of the first primer and a set of four distinguishably labeled primers comprising the tag sequences of the set of four downstream second primers.

As used herein, the term "sample" refers to a biological material which is isolated from its natural environment and containing a polynucleotide. A "sample" according to the invention can consist of purified or isolated polynucleotide, or it may comprise a biological sample such as a tissue sample, a biological fluid sample, or a cell sample comprising a polynucleotide. A biological fluid includes blood, plasma, sputum, urine, cerebrospinal fluid, lavages, and leukophoresis samples. A sample of the present invention may be any plant, animal, bacterial or viral material containing a polynucleotide.

As used herein, the term "polymorphism" refers to a nucleic acid sequence variation. When compared to a naturally occurring sequence, a polymorphism can be present at a frequency of greater than 0.01 %, 0.1 %, 1% or greater in a population. As used herein, a polymorphism can be an insertion, deletion, duplication, or rearrangement. As used herein, a "single nucleotide polymorphism" or "SNP" refers to nucleic acid sequence variation at a single nucleotide residue, including a single nucleotide deletion, insertion, or base change. A polymorphism, including a SNP, can be phenotypically neutral or can have an associated variant phenotype that distinguishes it from that exhibited by the predominant sequence at that locus. As used herein, "neutral polymorphism" refers to a polymorphism in which the sequence variation does not alter gene function, and "mutation" or "functional polymorphism" refers to a sequence variation which does alter gene function, and which thus has an associated phenotype.

When referring to the genotype of an individual with regard to an SNP, the "predominant allele" is that which occurs most frequently in the population being examined (i.e., when there are two alleles, the allele that occurs in greater than 50% of the population is the predominant allele; when there are more than two alleles, the "predominant allele" is that which occurs in the subject population at the highest frequency, e.g., at least 5% higher frequency, relative to the other alleles at that site). The term "variant allele" is used to refer to the allele or alleles occurring less frequently than the predominant allele in that population (e.g., when there are two alleles, the variant allele is that which occurs in less than 50% of the subject population; when there are more than two alleles, the variant alleles are all of those that occur less frequently, e.g., at least 5% less frequently, than the predominant allele).

As used herein, the term "polymorphic site" refers to the position, in a polymorphic nucleotide sequence, of the nucleotide that varies among individuals.

As used herein, an "oligonucleotide primer" refers to a polynucleotide molecule (i.e., DNA or RNA) capable of annealing to a polynucleotide template and providing a 3' end to produce an extension product which is complementary to the polynucleotide template. The conditions for initiation and extension usually include the presence of four different deoxyribonucleoside triphosphates and a polymerization-inducing agent such as DNA polymerase or reverse transcriptase, in a suitable buffer ("buffer" includes substituents which are cofactors, or which affect pH, ionic strength, etc.) and at a suitable temperature. The primer according to the invention may be single- or double-stranded. The primer is single-stranded for maximum efficiency in amplification, and the primer and its complement form a double-stranded polynucleotide. "Primers" useful in the present invention are less than or equal to 100 nucleotides in length, e.g., less than or equal to 90, or 80, or 70, or 60, or 50, or 40, or 30, or 20, or 15, or equal to 10 nucleotides in length.

As used herein, the term "polymerase extension" means the template-dependent incorporation of at least one complementary nucleotide, by a nucleic acid polymerase, onto the 3' end of an annealed primer. Polymerase extension preferably adds more than one nucleotide, preferably up to and including nucleotides corresponding to the full length of the template. Conditions for polymerase extension vary with the identity of the polymerase. The temperature of polymerase extension is based upon the known activity properties of the enzyme. In general, although the enzymes retain at least partial activity below their optimal extension temperatures, polymerase extension by the most commonly used thermostable polymerases (e.g., Taq polymerase and variants thereof) is performed at 65°C to 75°C, preferably about 68-72°C.

As used herein, the term "primer extension products" refers to nucleic acid molecules generated by the process of polymerase extension.

As used herein, the term "tag sequence," or simply "tag" refers to a nucleotide sequence, preferably a heterologous or artificial nucleotide sequence, that is attached to an oligonucleotide primer via standard phosphodiester linkage (i.e., phosphodiester linkage between the 3' OH of the tag and the 5' phosphate of the oligonucleotide) and permits the identification or tracing of polynucleotides into which the "tag" is incorporated (incorporated for example, by primer extension or amplification of a primer extension product). A "tag" sequence according to the invention will comprise at least 15, and preferably 20 to 30 nucleotides and will preferably not hybridize under primer extension conditions to a sequence in the genome of the organism being genotyped. A tag sequence according to the invention can be, but is not necessarily, random.

As used herein, the term "specifically corresponds" means that a given nucleic acid tag sequence on an oligonucleotide is only used with a given 3'-terminal nucleotide, such that the presence of the tag sequence is indicative of the presence of that 3'-terminal nucleotide. For example, tag sequence "1" would only be used on an oligonucleotide with a 3'-terminal A, tag sequence "2" would only be used on an oligonucleotide with a 3'-terminal C, tag sequence "3" would only be used on an oligonucleotide with a 3'-terminal G and tag sequence "4" would only be used on an oligonucleotide with a 3'-terminal T. Thus, in a method according to the invention, if a fragment amplifies with a primer specific for tag 2, it is known that the 3'-terminal nucleotide of the original primer extension primer was a C, and therefore, that the polymorphic nucleotide is a G in that sample.

As used herein, the term "amplification regimen" refers to a process of specifically amplifying, i.e., increasing the abundance of, a nucleic acid sequence of interest. An amplification regimen according to the invention comprises at least two, and preferably at least 5, 10, 15, 20, 25, 30, 35 or more iterative cycles, where each cycle comprises the steps of: 1) strand separation (e.g., thermal denaturation); 2) oligonucleotide primer annealing to template molecules; and 3) nucleic acid polymerase extension of the annealed primers. Conditions and times necessary for each of these steps are well known in the art. Amplification achieved using an amplification regimen is preferably exponential, but can alternatively be linear. An amplification regimen according to the invention is preferably performed in a thermal cycler, many of which are commercially available.

As used herein, the term "set" means a group of nucleic acid samples, primers or other entities. A set will comprise a known number of, and at least two of such entities.

As used herein, the term "subset" means a group comprised by a set as defined herein, wherein the subset group is less than every member of the set. A subset as used herein can consist of a single entity.

As used herein, the relative terms "upstream" and "downstream" are used to refer to positions on a polynucleotide relative to a polymorphic site. Generally, "upstream" refers to 5' of the polymorphic site, and "downstream" refers to 3' of the polymorphic site. It is understood that the choice of "upstream" and "downstream" in a double-stranded DNA sequence is largely arbitrary, in that one may choose to focus on either strand, and the direction that is "upstream" or "downstream" of the polymorphic site will change, depending upon which strand is chosen as the "reference" strand. In order to avoid any ambiguity, as used herein to describe a given method, the "reference" strand for the selection of the terms "upstream" and "downstream" will remain the same throughout that method.

As used herein, the term "distinguishably labeled" means that the signal from one labeled oligonucleotide primer or a nucleic acid molecule into which it is incorporated can be distinguished from the signal from another such labeled primer or nucleic acid molecule. Detectable labels can comprise, for example, a light-absorbing dye, a fluorescent dye, or a radioactive label. Fluorescent dyes are preferred. Generally, a fluorescent signal is distinguishable from another fluorescent signal if the peak emission wavelengths are separated by at least 20 nm. Greater peak separation is preferred, especially where the emission peaks of fluorophores in a given reaction are wide, as opposed to narrow or more abrupt peaks.

As used herein, the term "separating nucleic acid molecules" refers to the process of physically separating nucleic acid molecules in a sample or aliquot on the basis of size and/or charge. Electrophoretic separation is preferred, and capillary electrophoretic separation is most preferred.

As used herein, the term "detecting the incorporation" refers to the process of determining whether a given labeled oligonucleotide primer has been extended, thereby incorporating the label into the primer extension or amplification product. Detection can be by any means compatible with the detectable label, but will preferably involve detection of a fluorescent label. Detecting encompasses determination of both the presence and the abundance of label in a primer extension or amplification product. Fluorescence detectors are well known in the art.

As used herein, the term "specifically hybridizes" means that under given hybridization conditions a probe or primer hybridizes only to a target sequence in a sample comprising the target sequence. Given hybridization conditions include the conditions for the annealing step in an amplification regimen, i.e., annealing temperature selected on the basis of predicted Tₘ, and salt conditions suitable for the polymerase enzyme of choice.

As used herein, the term "strand separation" or "separating the strands" means treatment of a nucleic acid sample such that complementary double-stranded molecules are separated into two single strands available for annealing to an oligonucleotide primer. Strand separation according to the invention is achieved by heating the nucleic acid sample above its Tₘ. Generally, for a sample containing nucleic acid molecules in buffer suitable for a nucleic acid polymerase, heating to 94°C is sufficient to achieve strand separation according to the invention. An exemplary buffer contains 50 mM KCl, 10 mM Tric-HCl (pH 8.8@ 25°C), 0.5 to 3 mM MgCl₂, and 0.1% BSA.

As used herein, the term "primer annealing" or "re-annealing" means permitting oligonucleotide primers to hybridize to template nucleic acid strands. Conditions for primer annealing vary with the length and sequence of the primer and are based upon the calculated Tₘ for the primer. Generally, an annealing step in an amplification regimen involves reducing the temperature following the strand separation step to a temperature based on the calculated Tₘ for the primer sequence, for a time sufficient to permit such annealing. Tₘ can be readily predicted by one of skill in the art using any of a number of widely available algorithms (e.g., Oligo™, Primer Design and programs available on the internet, including Primer3 and Oligo Calculator). For most amplification regimens, the annealing temperature is selected to be about 5°C below the predicted Tₘ, although temperatures closer to and above the Tₘ (e.g., between 1°C and 5°C below the predicted Tₘ or between 1°C and 5°C above the predicted Tₘ) can be used, as can temperatures more than 5°C below or above the predicted Tₘ (e.g., 6°C below, 8°C below, 10°C below or lower and 6°C above, 8°C above, or 10°C above). Generally, the closer the annealing temperature is to the Tₘ, the more specific is the annealing. Time of primer annealing depends largely upon the volume of the reaction, with larger volumes requiring longer times, but also depends upon primer and template concentrations, with higher relative concentrations of primer to template requiring less time than lower. Depending upon volume and relative primer/template concentration, primer annealing steps in an amplification regimen can be on the order of 1 second to 5 minutes, but will generally be between 10 seconds and 2 minutes, preferably on the order of 30 seconds to 2 minutes.

As used herein, the term "3' region that hybridizes to a sequence at a known distance upstream of a known polymorphic site" refers to a sequence of nucleotides, located at the 3' end of an oligonucleotide, that specifically hybridize to a sequence upstream (i.e., 5') of a known polymorphic site being genotyped in a sample of nucleic acid. The "3' region that hybridizes" will be at least 12 nucleotides long, and preferably at least 15, 18, 21, 24, 27, 30 nucleotides or more. The "region that hybridizes" is selected to be a known distance from the polymorphic site so as to give rise to an amplification product that is distinctly sized relative to other amplification products in a method according to the invention. The "known distance" can be from 50 to 1000 nucleotides, and is preferably from 50 to 500 nucleotides or 50 to 250 nucleotides.

As used herein, a "region that hybridizes 3' of and adjacent to a polymorphic site" is an oligonucleotide sequence, generally 10 to about 25 nucleotides in length, that specifically hybridizes 3' of a polymorphic site, such that the penultimate 3' nucleotide of the region is hybridized one nucleotide downstream of the polymorphic site. The invention makes use of a set of four primers comprising such a region, with the set comprised of oligonucleotides having four different 3' terminal nucleotides, G, A, T or C, only one of which will hybridize to the nucleotide at the polymorphic site and permit primer extension by a nucleic acid polymerase.

As used herein, the term "variable 3'-terminal nucleotide" refers to a 3'-terminal nucleotide of an oligonucleotide that can be any of G, A, T or C.

As used herein, the term "opposite the polymorphic site" means that a nucleotide, the 3'-terminal nucleotide on an oligonucleotide primer hybridized to a polymorphism-containing nucleic acid strand, is positioned such that it will form a Watson-Crick hydrogen bonded base pair with the nucleotide at the polymorphic position if the 3'-terminal nucleotide is complementary to the nucleotide at the polymorphic site.

As used herein, the term "complementary" refers to the hierarchy of hydrogen-bonded base pair formation preferences between the four deoxyribonucleotides G, A, T, and C, such that A pairs with T and G pairs with C.

As used herein, the phrase "nucleic acid polymerase" refers an enzyme that catalyzes the template-dependent polymerization of nucleoside triphosphates to form primer extension products that are complementary to one of the nucleic acid strands of the template nucleic acid sequence. A nucleic acid polymerase enzyme initiates synthesis at the 3' end of an annealed primer and proceeds in the direction toward the 5' end of the template. Numerous nucleic acid polymerases are known in the art and commercially available. One group of preferred nucleic acid polymerases are thermostable, i.e., they retain function after being subjected to temperatures sufficient to denature annealed strands of complementary nucleic acids.

As used herein, the term "aliquot" refers to a sample of an amplification reaction taken during the cycling regimen. An aliquot is less than the total volume of the reaction, and is preferably 0.1-30 % in volume. In one embodiment of the invention, for each aliquot removed, an equal volume of reaction buffer containing reagents necessary for the reaction (e.g., buffer, salt, nucleotides, and polymerase enzyme) is introduced.

As used herein, the term "conditions that permit the extension of an annealed oligonucleotide such that extension products are generated" refers to the set of conditions including, for example temperature, salt and co-factor concentrations, pH, and enzyme concentration under which a nucleic acid polymerase catalyzes primer extension. Such conditions will vary with the identity of the nucleic acid polymerase being used, but the conditions for a large number of useful polymerase enzymes are well known to those skilled in the art. One exemplary set of conditions is 50 mM KCl, 10 mM Tric-HCl (pH 8.8@ 25°C), 0.5 to 3 mM MgCl₂, 200 µM each dNTP, and 0.1% BSA at 72 °C, under which Taq polymerase catalyzes primer extension.

As used herein, the term "real time" means that the measurement of the accumulation of products in a nucleic acid amplification reaction is at least initiated, and preferably completed during or concurrent with the amplification regimen. Thus, for the measurement process to be considered "real time", at least the initiation of the measurement or detection of amplification products in each aliquot is concurrent with the amplification process. By "initiated" is meant that an aliquot is withdrawn and placed into a separation apparatus, e.g., a capillary electrophoresis capillary, and separation is begun. The completion of the measurement is the detection of labeled species in the separated nucleic acids from the aliquot. Because the time necessary for separation and detection may exceed the time of each individual cycle of the amplification regimen, there may be a lag in the detection of the amplification products of up to 120 minutes beyond the completion of the amplification regimen. Preferably such lag or delay is less than 30 minutes, e.g., 25 minutes, 20 minutes, 15 minutes, 10 minutes, 5 minutes, 4 minutes, 3 minutes, 2 minutes, 1 minute or less, including no lag or delay.

As used herein, the term "capillary electrophoresis" means the electrophoretic separation of nucleic acid molecules in an aliquot from an amplification reaction wherein the separation is performed in a capillary tube. Capillary tubes are available with inner diameters from about 10 to 300 µm, and can range from about 0.2 cm to about 3 m in length, but are preferably in the range of 0.5 cm to 20 cm, more preferably in the range of 0.5 cm to 10 cm. In addition, the use of microfluidic microcapillaries (available, e.g., from Caliper or Agilent Technologies) is specifically contemplated within the meaning of "capillary electrophoresis."

As used herein, the term "modular apparatus" means an apparatus that comprises individual units in which certain processes of the methods according to the invention are performed. The individual units of a modular apparatus can be but are not necessarily physically connected, but it is preferred that the individual units are controlled by a central control device such as a computer. An example of a modular apparatus useful according to the invention has a thermal cycler unit, a sampler unit, and a capillary electrophoresis unit with a fluorescence detector. The modular apparatus useful according to the invention can also comprise a robotic arm to transfer samples from the cycling reaction to the electrophoresis unit.

As used herein, the term "sampling device" refers to a mechanism that withdraws an aliquot from an amplification during the amplification regimen. Sampling devices useful according to the invention will preferably be adapted to minimize contamination of the cycling reaction(s), by, for example, using pipeting tips or needles that are either disposed of after a single sample is withdrawn, or by incorporating one or more steps of washing the needle or tip after each sample is withdrawn. Alternatively, the sampling device can contact the capillary to be used for capillary electrophoresis directly with the amplification reaction in order to load an aliquot into the capillary. Alternatively, the sample device can include a fluidic line (e.g. a tube) connected to the controllable valve which will open at particular cycle. Sampling devices known in the art include, for example, the multipurpose Robbins Scientific Hydra 96 pipettor, which is adapted to sampling to or from 96 well plates. This and others can be readily adapted for use according to the methods of the invention.

As used herein, the term "robotic arm" means a device, preferably controlled by a microprocessor, that physically transfers samples, tubes, or plates containing samples from one location to another. Each location can be a unit in a modular apparatus useful according to the invention. An example of a robotic arm useful according to the invention is the Mitsubishi RV-E2 Robotic Arm. Software for the control of robotic arms is generally available from the manufacturer of the arm.

As used herein, the term "amplified product" refers to polynucleotides which are copies of a portion of a particular polynucleotide sequence and/or its complementary sequence, which correspond in nucleotide sequence to the template polynucleotide sequence and its complementary sequence. An "amplified product," according to the invention, may be DNA or RNA, and it may be double-stranded or single-stranded.

As used herein, the term "distinctly sized amplification product" means an amplification product that is resolvable from amplification products of different sizes. "Different sizes" refers to nucleic acid molecules that differ by at least one nucleotide in length. Generally, distinctly sized amplification products useful according to the invention differ by greater than or equal to more nucleotides than the limit of resolution for the separation process used in a given method according to the invention. For example, when the limit of resolution of separation is one base, distinctly sized amplification products differ by at least one base in length, but can differ by 2 bases, 5 bases, 10 bases, 20 bases, 50 bases, 100 bases or more. When the limit of resolution is, for example, 10 bases, distinctly sized amplification products will differ by at least 10 bases, but can differ by 11 bases, 15 bases, 20 bases, 30 bases, 50 bases, 100 bases or more.

As used herein, the term "profile" or the equivalent terms "amplification curve" and "amplification plot" mean a mathematical curve representing the signal from a detectable label incorporated into a nucleic acid sequence of interest at two or more steps in an amplification regimen, plotted as a function of the cycle number from which the samples were withdrawn. The profile is preferably generated by plotting the fluorescence of each band detected after capillary electrophoresis separation of nucleic acids in the individual reaction samples. Most commercially available fluorescence detectors are interfaced with software permitting the generation of curves based on the signal detected.

The number of genes that could be investigated in a single reaction can be estimated based on the measurable difference of the product size (1-2 bases) and on the separable size of PCR products (500-1000bp) and can be as high as 1000, but is preferably 100-200.

As used herein, the term "heat-labile exonuclease" refers to an enzyme that degrades single-stranded nucleic acid molecules or overhanging single strands on partially double stranded nucleic acid molecules and is irreversibly inactivated by incubation at an elevated temperature. The temperature for inactivation will vary with the enzyme and with, for example, buffer conditions and enzyme concentration. Conditions for enzyme inactivation are known to those skilled in the art. A non-limiting example of a heat-labile exonuclease useful according to the invention is Exonuclease I (ExoI), from E. coli (commerically available from, e.g., New England Biolabs, Beverly MA). ExoI is inactivated by incubation at 80°C for 20 minutes.

As used herein, the term "substantially lacking sequence specific for a gene in the genome of the organism" means that a given primer will not generate a primer extension product when incubated under primer extension conditions with genomic DNA from the organism being investigated with respect to polymorphisms.

### BRIEF DESCRIPTION OF THE FIGURES

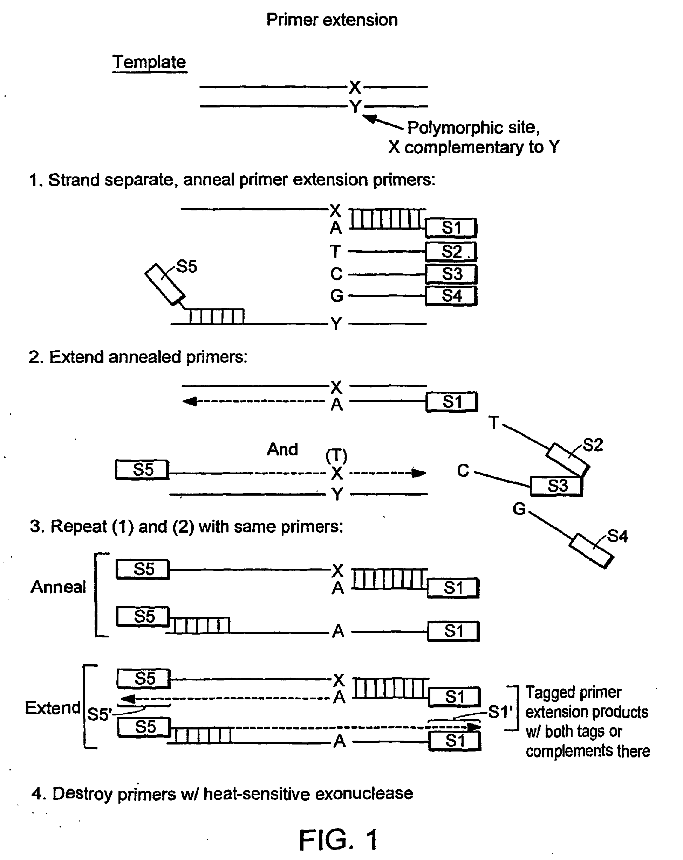

Figure 1 shows a schematic diagram of primer extension reactions useful in one embodiment of the invention. S1 and S5 are different sequence tags.

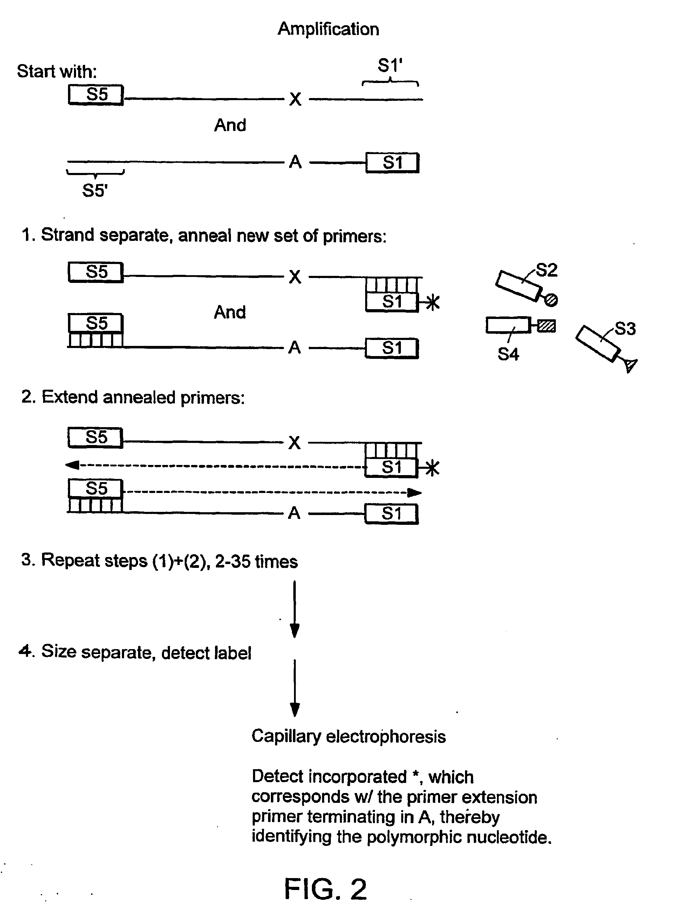

Figure 2 shows a schematic diagram of an amplification regimen and detection useful in one embodiment of the invention. S1 and S5 are tag sequence primers that differ from one another but are identical to S1 to SS shown in Figure 1.

### DETAILED DESCRIPTION OF THE INVENTION

The invention provides methods of determining the genotype of a nucleic acid sample with respect to known single nucleotide polymorphisms. The methods of the invention employ primer extension reactions that incorporate sequence tags permitting the simultaneous identification of the specific nucleotides present at a group of SNPs. Tagged fragments are then amplified using sets of primers specific for the tags wherein the downstream primer is labeled. During the amplification regimen, aliquots of the reaction are withdrawn and subjected to size separation and detection of the amplified fragments. The nucleotides present at the polymorphic sites are identified based on the size and identity of the label attached to the amplified fragments. Because both amplimer size and incorporated label are detected, the system is well suited for multiplexing. Further, the separation and detection are performed during the amplification reaction, such that a profile of the amplification reaction is generated in real time. The real time aspect provides rapid analysis as well as information regarding the course of the amplification that is useful in identifying and eliminating artifactual signals caused, for example, by interactions between primers.

### Generating sequence tagged primer extension products:

As a first step, the invention requires the generation of sequence-tagged primer extension products. A critical aspect of this step is that the tag on any particular extension product specifically corresponds with the identity of the nucleotide at the polymorphic site. In this step, the tag is incorporated by the extension of a primer with the following general structure:
5'- Tag_{c} - target complement - V_{c}-3'
wherein "Tag_{c}" is the tag sequence that corresponds with the identity of the nucleotide at the 3' terminus of the primer, "target complement" is the 3' region of the primer that specifically hybridizes adjacent to the known SNP, and V_{c} is a variable 3' terminal nucleotide that corresponds with the identity of the Tag_{c} sequence. The Tag_{c} sequence is preferably 20 to 30 nucleotides in length and preferably does not hybridize under primer extension conditions to a sequence in the genome of the organism being genotyped or to any of the other primers used in a given reaction. The "target complement" is long enough to provide specific hybridization between the primer and the sequence adjacent to a known SNP, and will generally be about 10 to 25 nucleotides in length. V_{c} is selected from dG, dA, dT and dC, and is positioned so that it is opposite the known polymorphic site when the primer is hybridized to the nucleic acid sample being interrogated. V_{c} will base pair with the nucleotide at the polymorphic site only if it is complementary to the nucleotide at that site. Because a nucleic acid polymerase, e.g., Taq polymerase, will only extend a primer if the 3'-terminal nucleotide is base paired with the adjacent nucleotide on the template strand, the extension of a primer with a known 3'-terminal nucleotide opposite the polymorphic site identifies the nucleotide present at the polymorphic site as the complement of the 3'-terminal nucleotide.

A set of downstream primer extension primers useful for the identification of an SNP will include four different tag sequences, one each to correspond to a 3'-terminal dG, dA, dT or dC. Thus, if the tags are referred to as Tags 1-4, for example, Tag 1 would be used on the primer terminating in a 3' dG, Tag 2 would be used on the primer terminating in a 3' dA, Tag 3 would be used on the primer terminating in a 3' dT, and Tag 4 would be used on the primer terminating in a 3' dC. A major advantage of the methods disclosed herein is that one can use the same set of four downstream Tag_{c} sequences in assays for multiple SNPs, because the resulting amplification products will differ in size. This limits the possibilities for non-template directed interprimer interactions in the amplification step that tend to interfere with multiplex amplifications.

Sequence-tagged upstream primers are used to generate the opposite strand of a given SNP-containing sequence. These primers will have the general structure:
5'-Tag - target complement - 3'
wherein "Tag" refers to a sequence tag different from each of those used in a downstream set of primer extension primers, and "target complement" refers to a sequence complementary to a region upstream of the known SNP. The "Tag" sequence on the upstream primer is preferably 20 to 30 nucleotides in length and preferably does not hybridize under primer extension conditions to a sequence in the genome of the organism being genotyped, or to any of the other primers being used in a given reaction. The "target complement" is long enough to provide specific hybridization under primer extension conditions between the primer and a sequence upstream of a known SNP, and will generally be about 10 to 25 nucleotides in length. The distance upstream will generally be at least 50 nucleotides, but can be 50 to 1000 nucleotides or more, preferably 50 to 500, or 50 to 250 nucleotides upstream of the polymorphic site. The distance of the upstream primer sequence from the polymorphic site determines the size or length of the later amplification products. The sizes of the later amplification products must be selected so as to differ by more than the resolution limit of the system used for size separation. Thus, if the limit of resolution of separation is one base, the sizes of the amplification products should be selected to differ by at least one base in length, and preferably more (e.g., at least 5, 10, 15 bases or more). When the limit of resolution is, for example, 10 bases, sizes of the amplification products should differ by at least 10 bases, and preferably more (e.g., at least 15, 20, 25, 30 bases or more).

The terms "upstream" and "downstream" are used herein in order to facilitate the description of the invention. However, it is recognized that because of the double-stranded nature of DNA, a polymorphism could be approached with SNP-specific primers from either side, that is, from upstream or downstream, by hybridization of the primer to one strand as opposed to the other. The invention specifically contemplates the interrogation of SNPs on either strand of the genomic DNA.

In order to generate sequence-tagged primer extension products according to the invention, a nucleic acid sample is denatured, preferably by heat, e.g., to 95°C for 2 minutes or more, and allowed to re-anneal in the presence of an upstream extension primer and a set of downstream primer extension primers for each SNP to be interrogated in the reaction. The denaturing and annealing is best performed in a buffer compatible with the nucleic acid polymerase to be used for the primer extension reaction, e.g., 1X Taq polymerase buffer. Re-annealing is performed at a temperature below the Tₘ of the primers, generally between about 20°C and 60°C, although lower or higher temperatures may be suitable for some primers. Primers should be present at about 15 to 500 nM for each primer. Optimal primer concentrations can be determined empirically by one of skill in the art with a minimum of experimentation, for example by setting up test reactions in which the primers are varied over the 15 to 500 nM range and analyzing the results with respect to the relative resolution, yield and specificity of the extension or amplification reactions.

Following annealing in the presence of the primers, polymerization is performed using a nucleic acid polymerase. Numerous polymerases sufficient for this step are known and can be selected by one skilled in the art. Among the most commonly used enzymes are the thermostable Taq polymerase and other thermostable polymerases, e.g., Pfu polymerase. Primer extension is performed under standard conditions for the enzyme chosen, e.g., 50 mM KCl, 10 mM Tric-HCl (pH 8.8@ 25°C), 0.5 to 3 mM MgCl₂, and 0.1% BSA and 100 µM each dNTP at 72°C for two minutes.

The first round of primer extension results in a population in which one strand has an upstream primer and tag sequence incorporated and the other strand has a downstream primer and tag sequence incorporated. The downstream primer incorporated for each SNP is the one in which the 3'-terminal nucleotide was complementary to the nucleotide at the polymorphic site on the target DNA. The incorporation of that downstream primer necessarily incorporates the tag sequence associated with or corresponding to that 3'-terminal nucleotide. In order to generate a population in which molecules representing each strand carry both an upstream tag or its complement and a downstream tag or its complement, the products of the first primer extension reaction are subjected to another round of denaturing, re-annealing in the presence of the same primers, and polymerase extension of those primers.

Following the second round of primer extension, non-extended primers are removed. Any method of primer removal can be used, e.g., electrophoresis or column chromatography, but it is preferred that a heat labile exonuclease specific for single-stranded DNA be used. The use of a heat-labile exonuclease avoids the need for time-consuming separation and purification procedures and the possibility for contamination or sample loss. Heat labile exonucleases useful according to the invention include, for example E. coli Exonuclease I (ExoI), and Exonuclease VII (ExoVII). ExoI, for example, is active at 37°C but is inactivated by incubation for 20 minutes at 80°C.

The primers used for primer extension are removed so that new primers, corresponding to the incorporated upstream and downstream tag sequences, can be used to amplify the primer extension products. Following the removal of the first primers, a set of primers comprising an upstream tag sequence primer and four downstream tag sequence primers is added. Each of the four downstream tag sequence primers is distinguishably labeled (e.g., end labeled) with a fluorescent dye. The mixture with the new primers added is then subjected to an amplification regimen comprising cycles of thermal denaturation, re-annealing and polymerase extension. The amplification regimen should comprise at least two cycles, but will preferably comprise 2 to 35 cycles, more preferably 10 to 30 cycles, and more preferably 15 to 25 cycles.

During the cycling regimen, following at least one of the cycles of denaturation, primer annealing and primer extension in this aspect of the invention, a sample or aliquot of the reaction is withdrawn from the tube or reaction vessel, and nucleic acids in the aliquot are separated and detected. The separation and detection are performed concurrently with the cycling regimen, such that a curve representing product abundance as a function of cycle number can be generated while the cycling occurs. As used herein, the term "concurrently" means that the separation is at least initiated while the cycling regimen is proceeding. Depending upon the separation technology used (e.g., capillary electrophoresis) and the number and size of species to be separated in a given reaction, the separation will most often require on the order of 1-120 minutes per aliquot. Thus, when separation steps take longer than the duration of each cycle, and when samples are withdrawn after, for example, every cycle, the separation steps will be completed after the completion of the full cycling regimen. However, as used herein, this situation is still considered to be "concurrent" separation, as long as the separation of each sample was initiated during the cycling regimen. Concurrent separation is most preferably performed through use of a robotic sampler that deposits the samples to the separation apparatus immediately after the samples are withdrawn from the cycling reaction.

In the manner described above, the identity of the nucleotide at a polymorphic site is determined by detection of the fluorescent signals on the size-separated amplification products. Because each of the four downstream tag primers is labeled with a distinguishable fluorescent label, and because the tag on a given primer corresponds to the identity of the 3'-terminal nucleotide of the original downstream primer extension primer, the incorporation and detection of that fluorescently labeled tag identifies the nucleotide at the polymorphic site.

In a preferred aspect, the original primer extension reactions include primer sets that recognize more than one SNP. In this aspect, each different polymorphism will be represented by a distinctly sized amplification product. For example, one can include additional upstream primers, each comprising the same tag sequence and varying in the 3' region that hybridizes at a distinct distance upstream of an additional known SNP. In concert with the additional upstream primer, each additional SNP to be interrogated requires a set of four downstream primer extension primers, each member of the set comprising in 5' to 3' order: a) a tag sequence that corresponds to the 3' terminal nucleotide of that primer, wherein the tag sequence is the same tag sequence that corresponds to that 3'-terminal nucleotide on the downstream primers used for other SNPs being interrogated in the same series of reactions; b) a region sufficient to direct specific hybridization of the primer downstream of and adjacent to a known SNP; and c) a variable 3'-terminal nucleotide that corresponds to the tag sequence on that primer, wherein when the primer is hybridized to its genomic target sequence, the 3'-terminal nucleotide is opposite the polymorphic site and can base pair with the nucleotide at that site if it is complementary. Following two primer extension reactions and the removal of non-incorporated primers as described above, a single amplification primer set is used, identical to that used when a single SNP is interrogated. That is, the amplification primer set will comprise an upstream primer comprising the upstream tag and a set of four distinguishably labeled primers comprising the four downstream tags on the primer extension primers, where the labels correspond to the tags that correspond to the nucleotides opposite the polymorphic site. The same amplification primer set can be used for each SNP interrogated because the incorporated tags are common between the sets. That is, all upstream primers have the same tag sequence, and all downstream primer extension primer sets have the same tag sequences corresponding to the same 3' terminal nucleotides. Each SNP interrogated will have a distinct size when separated, and the identity of the label incorporated into a molecule of that size positively identifies the nucleotide present at that polymorphic site. The ability to amplify and detect multiple SNPs with a single set of five amplification primers has the advantage of avoiding primer interaction problems prevalent when large numbers of primers are used for amplification. In addition, the effect of variations in primer annealing efficiency will be largely negated because all SNPs interrogated with a given amplification primer set will be affected by such variations to the same degree.

Further multiplexing can be achieved by using more than one set of five tag sequences. The additional sets will comprise tags distinct from those used in other sets. Care should be taken to avoid tags with complementarity to other tags to be used simultaneously. As above, each set will comprise upstream tags selected so that the amplification products are distinctly sized, and downstream tags in which the respective tags correspond to the 3'-terminal nucleotides of the primer extension primers. For the amplification, the downstream primers can be labeled with the same corresponding fluorescent labels as the other sets, or, preferably with a different set of distinguishable fluorescent labels. Following size separation, the amplified SNP-containing fragments are identified by size, and the identity of the nucleotide at the polymorphic site is identified by the label incorporated, as described above.

### General Considerations for Primer Design

Oligonucleotide primers are generally 5 to 100 nucleotides in length, preferably from 17 to 45 nucleotides, although primers of different lengths are of use. Primers for primer extension reactions are preferably 10 to 60 nucleotides long, while primers for amplification are preferably about 17-25 nucleotides in length. Primers useful according to the invention can be designed to have a particular melting temperature (Tₘ) by the method of melting temperature estimation. Commercial programs, including Oligo™, Primer Design and programs available on the internet, including Primer3 and Oligo Calculator can be used to calculate the Tₘ of a polynucleotide sequence useful according to the invention. Preferably, the Tₘ of an amplification primer useful according to the invention (e.g., a tag sequence), as calculated for example by Oligo Calculator, is between about 45°C and 65°C and more preferably between about 50°C and 60°C.

The Tₘ of a polynucleotide affects its hybridization to another polynucleotide (e.g., the annealing of an oligonucleotide primer to a template polynucleotide). In the methods of the invention, it is preferred that the oligonucleotide primers used in various steps selectively hybridize to a target template or to polynucleotides derived from the target template. Typically, selective hybridization occurs when two polynucleotide sequences are substantially complementary (at least about 65% complementary over a stretch of at least 14 to 25 nucleotides, preferably at least about 75%, more preferably at least about 90% complementary). See Kanehisa, M., 1984, Polynucleotide Res. 12: 203. As a result, it is expected that a certain degree of mismatch at the priming site is tolerated. Such mismatch may be small, such as a mono-, di- or tri-nucleotides. Alternatively, a region of mismatch may encompass loops, which are defined as regions in which there exists a mismatch in an uninterrupted series of four or more nucleotides.

Numerous factors influence the efficiency and selectivity of hybridization of the primer to a second polynucleotide molecule. These factors, which include primer length, nucleotide sequence and/or composition, hybridization temperature, buffer composition and potential for steric hindrance in the region to which the primer is required to hybridize, will be considered when designing oligonucleotide primers according to the invention.

A positive correlation exists between primer length and both the efficiency and accuracy with which a primer will anneal to a target sequence. In particular, longer sequences have a higher melting temperature (T_{M}) than do shorter ones, and are less likely to be repeated within a given target sequence, thereby minimizing promiscuous hybridization. Primer sequences with a high G-C content or that comprise palindromic sequences tend to self-hybridize, as do their intended target sites, since unimolecular, rather than bimolecular, hybridization kinetics are generally favored in solution. However, it is also important to design a primer that contains sufficient numbers of G-C nucleotide pairings since each G-C pair is bound by three hydrogen bonds, rather than the two that are found when A and T bases pair to bind the target sequence, and therefore forms a tighter, stronger bond. Hybridization temperature varies inversely with primer annealing efficiency, as does the concentration of organic solvents, e.g. formamide, that might be included in a priming reaction or hybridization mixture, while increases in salt concentration facilitate binding. Under stringent annealing conditions, longer hybridization probes or synthesis primers hybridize more efficiently than do shorter ones, which are sufficient under more permissive conditions. Preferably, stringent hybridization is performed in a suitable buffer (for example, 1X Taq Polymerase Buffer, or other buffer suitable for enzymes used for primer extension and amplification) under conditions that allow the polynucleotide sequence to hybridize to the oligonucleotide primers. Stringent hybridization conditions can vary (for example from salt concentrations of less than about 1M, more usually less than about 500 mM and preferably less than about 200 mM) and hybridization temperatures can vary (for example, from as low as 0°C to greater than 22°C, greater than about 30°C, and (most often) in excess of about 37°C) depending upon the lengths and/or the polynucleotide composition or the oligonucleotide primers. Longer fragments may require higher hybridization temperatures for specific hybridization. As several factors affect the stringency of hybridization, the combination of parameters is more important than the absolute measure of a single factor.

Unlike the design of primers made to recognize a sequence anywhere on a given gene, primers designed to hybridize near a known SNP are limited with respect to the modifications one can make to manipulate Tₘ. For example, where one would normally be able to shift up- or downstream on a sequence to find a region with a more favorable GC content, when a primer is designed to hybridize adjacent to a SNP, one cannot move the primer to another location. In this situation, then, the primary means of manipulating Tₘ is to vary the length of the complementary sequence in the primer.

### Sequence tags useful according to the invention:

Tags useful according to the invention are preferably heterologous or artificial nucleotide sequences of at least 15, and preferably 20 to 30 nucleotides in length. A tag will preferably not hybridize under PCR annealing conditions to a sequence in the genome of the organism being genotyped. A tag sequence according to the invention can be, but is not necessarily random. One can determine whether a potential tag sequence hybridizes under PCR annealing conditions to a sequence in the genome of an organism by using the tag sequence as a labeled primer in a primer extension reaction with genomic DNA from the organism of interest as template. The labeled primer is annealed to the genomic DNA at the annealing temperature one plans to use for the amplification steps of the method of the invention, and then incubated with thermostable polymerase under extension conditions. The reaction products are then electrophoretically separated alongside labeled probe alone. If the labeled tag appears in a band or bands larger than the tag primer, the tag primer hybridized under PCR annealing conditions to a sequence in the genome of the organism being genotyped. Care should also be taken to avoid tags with complementarity to other tags intended for use in the same reaction.

### Labeling_of oligonucleotide primers

Oligonucleotide primers useful according to the invention can be labeled, as described below, by incorporating moieties detectable by spectroscopic, photochemical, biochemical, immunochemical, enzymatic or chemical means. The method of linking or conjugating the label to the oligonucleotide primer depends, of course, on the type of label(s) used and the position of the label on the primer (i.e., 3'-terminal, 5'-terminal or body-labeled).

While fluorescent dyes are preferred, a variety of labels that would be appropriate for use in the invention, as well as methods for their inclusion in the primer, are known in the art and include, but are not limited to, enzymes (e.g., alkaline phosphatase and horseradish peroxidase) and enzyme substrates, radioactive atoms, chromophores, fluorescence quenchers, chemiluminescent labels, and electrochemiluminescent labels, such as Origen™ (Igen), that may interact with each other to enhance, alter, or diminish a signal. Of course, if a labeled molecule is used in a PCR based amplification assay involving thermal cycling, the label must be able to survive the temperature cycling required in this automated process. Ideally, four distinguishable labels that can be detected using similar equipment, methods and/or substrates are preferred. Fluorophores for use as labels in constructing labeled primers of the invention include, but are not limited to rhodamine and derivatives (such as Texas Red), fluorescein and derivatives (such as 5-bromomethyl fluorescein), Cy5, Cy3, JOE, FAM, Oregon Green^{™}, Lucifer Yellow, IAEDANS, 7-Me₂N-coumarin-4-acetate, 7-OH-4-CH₃-coumarin-3-acetate, 7-NH₂-4-CH₃-coumarin-3-acetate (AMCA), monobromobimane, pyrene trisulfonates, such as Cascade Blue, and monobromorimethyl-ammoniobimane. In general, fluorophores with wide Stokes shifts are preferred, to allow using fluorimeters with filters rather than a monochromometer and to increase the efficiency of detection.

The labels can be attached to the oligonucleotide directly or indirectly by a variety of techniques. Depending on the precise type of label or tag used, the label can be located at the 5' end of the primer or located internally in the primer, or attached to spacer arms of various sizes and compositions to facilitate signal interactions. 5' end labeling is preferred Using commercially available phosphoramidite reagents, one can produce oligomers containing functional groups (e.g., thiols or primary amines) at the 5'- terminus via an appropriately protected phosphoramidite, and can label them using protocols described in, for example, PCR Protocols: A Guide to Methods and Applications, Innis et al., eds. Academic Press, Ind., 1990.

Methods for introducing oligonucleotide functionalizing reagents to introduce one or more sulfhydryl, amino or hydroxyl moieties into the oligonucleotide primer sequence, typically at the 5' terminus, are described in U.S. Patent No. 4,914,210. A 5' phosphate group can be introduced as a radioisotope by using polynucleotide kinase and gamma-³²P-ATP or gamma-³³P-ATP to provide a reporter group. Biotin can be added to the 5' end by reacting an aminothymidine residue, or a 6-amino hexyl residue, introduced during synthesis, with an N-hydroxysuccinimide ester of biotin.

### Amplification

PCR methods are well-known to those skilled in the art, such as those described in Mullis and Faloona, 1987, Methods Enzymol., 155: 335, Saiki et al., 1985, Science 230:1350, and U.S. Patent Nos. 4,683,202, 4,683,195 and 4,800,159. In its simplest form, PCR is an *in vitro* method for the enzymatic synthesis of specific DNA sequences, using two oligonucleotide primers that hybridize to opposite strands and flank the region of interest in the target DNA. A repetitive series of reaction steps involving template denaturation, primer annealing and the extension of the annealed primers by DNA polymerase results in the exponential accumulation of a specific fragment whose termini are defined by the 5' ends of the primers. PCR is reported to be capable of producing a selective enrichment of a specific DNA sequence by a factor of 10⁹.

The length and temperature of each step of a PCR cycle, as well as the number of cycles, are adjusted according to the stringency requirements in effect. Annealing temperature and timing are determined both by the efficiency with which a primer is expected to anneal to a template and the degree of mismatch that is to be tolerated. The ability to optimize the stringency of primer annealing conditions is well within the knowledge of one of skill in the art. An annealing temperature between 20°C and 72°C is most commonly used. Initial denaturation of the template molecules is normally achieved by incubation at 92°C to 99°C for 4 minutes, followed by 20-40 cycles consisting of denaturation (94°C for 15 seconds to 1 minute), annealing (temperature based on Tₘ as discussed above, usually about 5°C below the Tₘ of the oligonucleotide in the reaction with the lowest Tₘ; usually 1-2 minutes), and extension (usually 72°C for 1-3 minutes).

### Sampling

Sampling during the amplification regimen can be performed at any frequency or in any pattern desired. It is preferred that sampling occurs after each cycle in the regimen, although less frequent sampling can also be used, for example, every other cycle, every third cycle, every fourth cycle, etc. While a uniform sample interval will most often be desired, there is no requirement that sampling be performed at uniform intervals. As just one example, the sampling routine may involve sampling after every cycle for the first five cycles, and then sampling after every other cycle.

Sampling can be as simple as manually pipetting an aliquot from the reaction, but is preferably automated such that the aliquot is automatically withdrawn at predetermined sampling intervals. It is preferred that the reaction mixture is replenished at each withdrawal with equal volumes of fresh components such as dNTPs, primers and DNA polymerase. For this and other aspects of the invention, it is preferred, although not necessary that the cycling be performed in a microtiter or multiwell plate format. This format, which uses plates comprising multiple reaction wells, not only increases the throughput of the assay process, but is also well adapted for automated sampling steps due to the modular nature of the plates and the uniform grid layout of the wells on the plates. Common microtiter plate designs useful according to the invention have, for example 12, 24, 48, 96, 384 or more wells, although any number of wells that physically fit on the plate and accommodate the desired reaction volume (usually 10-100 µl) can be used according to the invention. Generally, the 96 or 384 well plate format is preferred.

An automated sampling process can be readily executed as a programmed routine and avoids both human error in sampling (i.e., error in sample size and tracking of sample identity) and the possibility of contamination from the person sampling. Robotic samplers capable of withdrawing aliquots from thermal cyclers are available in the art. For example, the Mitsubishi RV-E2 Robotic Arm can be used in conjunction with a SciClone^{™} Liquid Handler or a Robbins Scientific Hydra 96 pipettor.

The robotic sampler useful according to the invention can be integrated with the thermal cycler, or the sampler and cycler can be modular in design. When the cycler and sampler are integrated, thermal cycling and sampling occur in the same location, with samples being withdrawn at programmed intervals by a robotic sampler. When the cycler and sampler are modular in design, the cycler and sampler are separate modules. In one embodiment, the assay plate is physically moved, e.g., by a robotic arm, from the cycler to the sampler and back to the cycler.

The volume of an aliquot removed at the sampling step can vary, depending, for example, upon the total volume of the amplification reaction, the sensitivity of product detection, and the type of separation used. Amplification volumes can vary from several microliters to several hundred microliters (e.g., 5 µl, 10 µl, 20 µl, 40 µl, 60 µl, 80 µl, 100 µl, 120 µl, 150 µl, or 200 µl or more), preferably in the range of 10-150 µl, more preferably in the range of 10-100 µl. Aliquot volumes can vary from 0.1 to 30 % of the reaction mixture.

### Separation of nucleic acids

Separation of nucleic acids according to the invention can be achieved by any means suitable for separation of nucleic acids, including, for example, electrophoresis, HPLC or mass spectrometry. Due to its speed and resolution, separation is preferably performed by capillary electrophoresis (CE).

CE is an efficient analytical separation technique for the analysis of minute amounts of sample. CE separations are performed in a narrow diameter capillary tube, which is filled with an electrically conductive medium termed the "carrier electrolyte." An electric field is applied between the two ends of the capillary tube, and species in the sample move from one electrode toward the other electrode at a rate which is dependent on the electrophoretic mobility of each species, as well as on the rate of fluid movement in the tube. CE may be performed using gels or liquids, such as buffers, in the capillary. In one liquid mode, known as "free zone electrophoresis," separations are based on differences in the free solution mobility of sample species. In another liquid mode, micelles are used to effect separations based on differences in hydrophobicity. This is known as Micellar Electrokinetic Capillary Chromatography (MECC).

CE separates nucleic acid molecules on the basis of charge, which effectively results in their separation by size or number of nucleotides. When a number of fragments are produced, they will pass the fluorescence detector near the end of the capillary in ascending order of size. That is, smaller fragments will migrate ahead of larger ones and be detected first.

CE offers significant advantages of over conventional electrophoresis, primarily in the speed of separation, small size of the required sample (on the order of 1-50 nl), and high resolution. For example, separation speeds using CE can be 10 to 20 times faster than conventional gel electrophoresis, and no post-run staining is necessary. CE provides high resolution, separating molecules in the range of about 10-1,000 base pairs differing by as little as a single base pair. High resolution is possible in part because the large surface area of the capillary efficiently dissipates heat, permitting the use of high voltages. In addition, band broadening is minimized due to the narrow inner diameter of the capillary. In free-zone electrophoresis, the phenomenon of electroosmosis, or electroosmotic flow (EOF) occurs. This is a bulk flow of liquid that affects all of the sample molecules regardless of charge. Under certain conditions EOF can contribute to improved resolution and separation speed in free-zone CE.

CE can be performed by methods well known in the art, for example, as disclosed in U.S. Patent Nos. 6,217,731; 6,001,230; and 5,963,456.
High throughput CE equipment is available commercially, for example, the HTS9610 High Throughput Analysis System and SCE 9610 fully automated 96-capillary electrophoresis genetic analysis system from Spectrumedix Corporation (State College, PA). Others include the P/ACE 5000 series from Beckman Instruments Inc (Fullerton, CA) and the ABI PRISM 3100 genetic analyzer (Applied Biosystems, Foster City, CA). Each of these devices comprises a fluorescence detector that monitors the emission of light by molecules in the sample near the end of the CE column. The standard fluorescence detectors can distinguish numerous different wavelengths of fluorescence emission, providing the ability to detect multiple fluorescently labeled species in a single CE run from an amplification sample.

Another means of increasing the throughput of the CE separation is to use a plurality of capillaries, or preferably an array of capillaries. Capillary Array Electrophoresis (CAE) devices have been developed with 96 capillary capacity (e.g., the MegaBACE instrument from Molecular Dynamics) and higher, up to and including even 1000 capillaries. In order to avoid problems with the detection of fluorescence from DNA caused by light scattering between the closely juxtaposed multiple capillaries, a confocal fluorescence scanner can be used (Quesada et al., 1991, Biotechniques 10:616-25).

The apparatus for separation (and detection) can be separate from or integrated with the apparatus used for thermal cycling and sampling. Because according to the invention the separation step is initiated concurrently with the cycling regimen, samples are preferably taken directly from the amplification reaction and placed into the separation apparatus so that separation proceeds concurrently with amplification. Thus, while it is not necessary, it is preferred that the separation apparatus is integral with the thermal cycling and sampling apparatus. In one embodiment, this apparatus is modular, comprising a thermal cycling module and a separation/detection module, with a robotic sampler that withdraws sample from the thermal cycling reaction and places it into the separation/detection apparatus.

### Detection

Amplification product detection methods useful according to the invention measure the intensity of fluorescence emitted by labeled primers when they are irradiated with light within the excitation spectrum of the fluorescent label. Fluorescence detection technology is highly developed and very sensitive, with documented detection down to a single molecule in some instances. High sensitivity fluorescence detection is a standard aspect of most commercially-available plate readers, microarray detection set-ups and CE apparatuses. For CE equipment, fiber optic transmission of excitation and emission signals is often employed. Spectrumedix, Applied Biosystems, Beckman Coulter and Agilent each sell CE equipment with fluorescence detectors sufficient for the fluorescence detection necessary for the methods described herein.

The fluorescence signals from two or more different fluorescent labels can be distinguished from each other if the peak wavelengths of emission are each separated by 20 nm or more in the spectrum. Generally the practitioner will select fluorophores with greater separation between peak wavelengths, particularly where the selected fluorophores have broad emission wavelength peaks. It follows that the more different fluorophores one wishes to include and detect concurrently in a sample, the narrower should be their emission peaks.

### EXAMPLES

### Example 1. Detection of single nucleotide differences.

Leber's hereditary optic neuropathy (LHON) is associated with the presence of several point mutations in mitochondrial DNA, at positions 3460, 11778 and 14459.

| Mutant: | SNP region (Polymorphic site shown in BOLD, underline) |
|---|---|
| 3460 | 5'-CGG GCT ACT ACA ACC CTT CGC TGA C**G**C CAT AAA-3' (SEQ ID NO: 1) |
| 11778 | 5'-TCA AAC TAC GAA CGC ACT CAC AGT C**G**C ATC ATA-3' (SEQ ID NO: 2) |
| 14459 | 5'-CTC AGG ATA CTC CTC AAT AGC CAT C**G**C TGT AGT-3' (SEQ ID NO: 3) |

The genotype of an individual with respect to SNPs in human mitochondrial DNA associated with Leber's hereditary optic neuropathy (LHON) can be determined as follows.

### Primer Extension:

### Primers:

### a) Upstream primers.

The upstream primers are as follows:

| Mutant | Upstream primer (tag sequences are in lower case) |
|---|---|
| 3460 | 5'-gttacaagat tctcacacgc taagg-TTC ATA GTA GAA GAG CGA TGG-3' (SEQ ID NO: 4) |
| 11778 | 5'-gttacaagat tctcacacgc taagg-AAA AAG CTA TTA GTG GGA GTA-3' |
| | (SEQ ID NO: 5) |
| 14459 | 5'-gttacaagat tctcacacgc taagg-TCG GGT GTG TTA TTA TTC TGA-3' (SEQ ID NO: 6) |

### b) Downstream primers.

The downstream primers are as follows:

| Mutant | | Downstream Primer |
|---|---|---|
| 3460 | G-primer: | 5'-agttggcgaa gcagtcgcta gaagaCGG GCT ACT ACA ACC CTT CGC TGA CG-3' (SEQ ID NO: 7) |
| | A-primer: | 5'-gatgctggtg tggctggtgt tcccgCGG GCT ACT ACA ACC CTT CGC TGA CA-3' (SEQ ID NO: 8) |
| | T-primer: | 5'-ggttggttgc acactggaga tattggCGG GCT ACT ACA ACC CTT CGC TGA CT-3' (SEQ ID NO: 9) |
| | C-primer: | 5'-ctggagcatc tggaaaagta gtaccCGG GCT ACT ACA ACC CTT CGC TGA CC-3' (SEQ ID NO: 10) |
| | | |
| 11778 | G-primer: | 5'-agttggcgaa gcagtcgcta gaagaTCA AAC TAC GAA CGC ACT CAC AGT CG-3' (SEQ ID NO: 11) |
| | A-primer: | 5'-gatgctggtg tggctggtgt tcccgTCA AAC TAC GAA CGC ACT CAC AGT CA-3' (SEQ ID NO: 12) |
| | T-primer: | 5'-ggttggttgc acactggaga tattggTCA AAC TAC GAA CGC ACT CAC AGT CT-3' (SEQ ID NO: 13) |
| | C-primer: | 5'-ctggagcatc tggaaaagta gtaccTCA AAC TAC GAA CGC ACT CAC AGT CC-3' (SEQ ID NO: 14) |
| 14459 | G-primer: | 5'-agttggcgaa gcagtcgcta gaagaCTC AGG ATA CTC CTC AAT AGC CAT CG-3' (SEQ ID NO: 15) |
| | A-primer: | 5'-gatgctggtg tggctggtgt tcccgCTC AGG ATA CTC CTC AAT AGC CAT CA-3' (SEQ ID NO: 16) |
| | T-primer: | 5'-ggttggttgc acactggaga tattggCTC AGG ATA CTC CTC AAT AGC CAT CT-3' (SEQ ID NO: 17) |
| | C-primer: | 5'-ctggagcatc tggaaaagta gtaccCTC AGG ATA CTC CTC AAT AGC CAT CC-3' (SEQ ID NO: 18) |

The full set of 5 primer extension primers for each polymorphic site (40 pmol each, 15 primers in total) is mixed with 1 µg of template genomic DNA from the individual to be tested, in 1X Pfu buffer (20 mM Tris-HCl, pH 8.8, 10 mM KCl, 10 mM (NH₄)₂SO₄, 2 mM MgSO₄, 0.1% Triton-X-100 and 0.1 mg/ml nuclease-free BSA) in a total volume of 50 µl. The mixture is heated to 94°C for 2 minutes and slowly cooled to room temperature, to permit primer annealing. 1 µl (2.5 U/µl) of cloned Pfu polymerase plus 1.25 µl of each dNTP (final concentration 200 µM) is added, and the sample is incubated at 72°C for 3 minutes. The sample is then cycled to 94°C for 2 minutes, then 50°C for 1 minute, and 72°C for 3 minutes to generate a population of primer extension products with an upstream primer or its complement and a downstream primer or its complement.

Primer extension primers are removed by the addition of 20 U of E. coli Exonuclease I (ExoI; New England Biolabs) and incubation at 37°C for 20 minutes. ExoI is then inactivated by incubation at 80°C for 20 minutes.

### Amplification:

After removal of primer extension primers, the 5 amplification primers (40 pmol of each primer in 1X Pfu buffer, final volume 75 µl) are added as follows:
a) Upstream Primer: 5'-gttacaagat tctcacacgc taagg-3' (SEQ ID NO: 19
b) Downstream primers: (distinguishably labeled)
   G-primer: 5'-R6G-agttggcgaa gcagtcgcta gaaga-3' (SEQ ID NO: 20)
   A-primer: 5'-FAM-gatgctggtg tggctggtgt tcccg-3' (SEQ ID NO: 21)
   T-primer: 5'-ROX-ggttggttgc acactggaga tattgg-3' (SEQ ID NO: 22)
   C-primer: 5'-JOE ctggagcatc tggaaaagta gtacc-3' (SEQ ID NO: 23)

Amplification is performed by adding 1 µl of fresh, cloned Pfu polymerase and cycling the reaction as follows: 35 cycles of 94°C for 45 sec., 50°C for 45 sec., and 72°C for 2 min. After each cycle, or at any chosen interval, an aliquot (0.5 µl) is withdrawn and loaded onto a prepared capillary electrophoresis apparatus. Separation is initiated and conducted during the amplification regimen. Amplified primer extension products are detected by fluorescence after separation over the length of the capillary. The signal strength of each fragment can be plotted for each cycle, to generate an amplification profile.

Amplified products are:

| Mutant | Product size | Wild-type polymorphic nucleotide | Mutant polymorphic nucleotide |
|---|---|---|---|
| 3460 | 249 | G | A (detected by ROX dye on 249 bp product) |
| 11778 | 350 | G | A (detected by ROX dye on 350 bp product) |
| 14459 | 456 | G | A (detected by ROX dye on 456 bp product) |

The method detailed in this example can be further multiplexed by including an additional upstream primer extension primer for each additional SNP, having the same upstream tag and a 3' region specific for a different SNP-containing fragment of a distinct size from those already included. Each additional SNP interrogated must also have its own set of 4 downstream primers carrying the same set of 4 downstream primer tags, a 3' region that specifically hybridizes adjacent to the SNP, and a variable 3'-terminal nucleotide that corresponds to the tag sequence.

Further multiplexing can be achieved by including new primer sets with a different set of upstream and downstream tags as described herein above.

## Claims

1. A method of determining, for a given nucleic acid sample, the identities of the nucleotides at a set of known polymorphic sites to be interrogated, said method comprising:
(a) subjecting to an amplification regimen, a population of primer extension products generated from a nucleic acid sample, each primer extension product comprising a member of a set of tag sequences, which tag sequence specifically corresponds to the presence of one specific nucleotide at a known polymorphic site, wherein said amplification regimen is performed using one upstream amplification primer for each sequence comprising a known polymorphic site to be interrogated, and a set of distinguishably labelled downstream amplification primers, each member of said set of downstream amplifications primers comprising a said tag sequence comprised by a member of said population of primer extension products and a distinguishable label that specifically corresponds to the presence of a specific nucleotide at said polymorphic site, and wherein said upstream amplification primers are selected such that each polymorphic site of said set of known polymorphic sites to be interrogated corresponds to a distinctly sized amplification product;
(b) separating nucleic acid molecules made during said amplification regimen by size and/or by charge;
(c) detecting incorporation of a distinguishably labeled downstream amplification primer in distinctly sized amplification products of step (a), thereby to determine the identity of the nucleotide at each said polymorphic site.

2. The method of claim 1, wherein each primer extension product comprises a member of a set of first tag sequences or its complement and a second tag sequence or its complement, the presence of which second tag sequence or its complement specifically corresponds to the presence of one specific nucleotide at a known polymorphic site, wherein for each polymorphic site in said set of polymorphic sites, said first tag sequence is located at a distinct distance 5' of said polymorphic site, relative to the distance of said first tag sequence from a polymorphic site on molecules in said sample containing other polymorphic sites, wherein said amplification regimen is performed using an upstream amplification primer comprising said first tag sequence, and a set of distinguishably labeled downstream amplification primers, each member of said set of downstream amplification primers comprising a said second tag sequence comprised by a member of said population of primer extension products and a distinguishable label that specifically corresponds to the presence of a specific nucleotide at said polymorphic site, and wherein said upstream amplification primers are selected such that each polymorphic site of said set of known polymorphic sites to be interrogated corresponds to a distinctly sized amplification product.

3. The method of claim 1, wherein said distinguishable label is a fluorescent label.

4. The method of claim 1, wherein said separating comprises capillary electrophoresis.

5. The method of claim 1, wherein said amplification regimen comprises at least two amplification reaction cycles, wherein each cycle comprises the steps of: (1) nucleic acid strand separation; (2) oligonucleotide primer annealing; and (3) polymerase extension of annealed primers.

6. The method of claim 5 further comprising the steps, during said amplification regimen and after at least one of said reaction cycles, of removing an aliquot of said amplification reaction, separating nucleic acid molecules by size and/or by charge, and detecting the incorporation of a said distinguishable label, wherein said detecting determines the identity of the nucleotide at said polymorphic site.

7. The method of claim 6, wherein said removing, separating and detecting are performed after each cycle in said regimen.

8. The method of claim 6, wherein said separating comprises capillary electrophoresis.

9. The method of claim 1, wherein steps (a) and (b) are performed in a modular apparatus comprising a thermal cycler, a sampling device, a capillary electrophoresis device and a fluorescence detector.

10. The method of claim 1, wherein said tag sequence comprises 15 to 40 nucleotides.

11. The method of claim 1, wherein said set of distinguishably labelled downstream amplification primers consists of: a subset that comprises a tag sequence that specifically corresponds to the presence of A at the polymorphic site; a subset that comprises a tag sequence that specifically corresponds to the presence of C at the polymorphic site; a subset that comprises a tag sequence that specifically corresponds to the presence of G at the polymorphic site; and a subset that comprises a tag sequence that specifically corresponds to the presence of T at the polymorphic site.

12. The method of claim 1 wherein said set of distinguishably labelled downstream amplification primers consists of a pair of oligonucleotides, one comprising a tag sequence that specifically corresponds to a first allele of the polymorphic site and one comprising a tag sequence that specifically corresponds to a second allele of the polymorphic site.

13. The method of claim 1, further comprising the step, before step (a), of removing primers not incorporated when said population of primer extension products were made.

14. The method of claim 13, wherein said step of removing comprises degrading said primers not incorporated when said population of primer extension products were made.

15. The method of claim 14, wherein said degrading is performed using a heat labile exonuclease.

16. The method of claim 15, wherein said heat labile exonuclease is selected from the group consisting of Exonuclease I and Exonuclease VII.

17. The method of claim 16, wherein said heat labile exonuclease is thermally inactivated before continuing to step (a).

18. The method of claim 1 wherein said population of primer extension products is generated by a method comprising:
I) providing a nucleic acid sample comprising said group of polymorphic sites;
II) separating the strands of said nucleic acid sample and re-annealing in the presence of:
a) a set of first oligonucleotide primers each comprising a 3' region that hybridizes to a sequence at a known distance upstream of a known polymorphic site, each member of said set of first oligonucleotide primers comprising a common sequence tag located 5' of said 3' region, and each member of said set of first oligonucleotide primers selected such that a distinctly sized amplification product is generated for each polymorphic site in said group of known polymorphic sites; and
b) a set of downstream amplification primers comprising, in 5' to 3' order:
i) a sequence tag selected from the group consisting of a tag specifically corresponding to G as the 3'-terminal nucleotide of said primer; a tag specifically corresponding to A as the 3'-terminal nucleotide of said primer, and a tag specifically corresponding to T as the 3'-terminal nucleotide of said primer; and a tag specifically corresponding to C as the 3'-terminal nucleotide of said primer;
ii) a region that specifically hybridizes to a sequence adjacent to and 3' of a polymorphic site in said group of polymorphic sites, wherein said set of downstream amplification primers comprises a subset of primers comprising a region that specifically hybridizes adjacent to said polymorphic site for each polymorphic site in said group of polymorphic sites; and
iii) a 3' terminal nucleotide selected from G, A, T or C, wherein said terminal nucleotide specifically corresponds to the sequence tag described in (i) on that downstream amplification primer, and wherein when said downstream amplification primer is hybridized to said sequence adjacent to and 3' of a polymorphic site, said 3'terminal nucleotide is opposite and polymorphic site;
III) contacting the annealed oligonucleotides resulting from step (II) with a nucleic acid polymerase under conditions that permit the extension of an annealed oligonucleotid such that extension products are generated, wherein the primer extension product from the first oligonucleotide primer, when separated from its complement, can serve as a template for the synthesis of the extension product of as a member of the set of second oligonucleotide primers, and vice versa;
IV) repeating strand separating and contacting steps (II) and (III) two times, such that a reaction mixture comprising a population of nucleic acid molecules is generated that comprises both a sequence identical to or complementary to said first oligonucleotide and a sequence identical to or complementary to a member of said set of downstream amplification primers;
V) contacting the population generated in step (IV) with a heat-labile exonuclease under conditions permitting the degradation of non-annealed oligonucleotide primers, such that non-annealed primers are degraded;
VI) thermally inactivating said heat-labile exonuclease; and wherein said step of subjecting a population of primer extension products to an amplification regimen is performed using an upstream amplification primer comprising the common sequence tag comprised by said first oligonucleotide primer,
and a set of downstream amplification primers, each member of said set of downstream amplification primers comprising a tag comprised by a member of said set of second oligonucleotide primers and a distinguishable label.

19. The method of claim 18, wherein said distinguishable label is a fluorescent label.

20. The method of claim 18, wherein said separating comprises capillary electrophoresis.

21. The method of claim 18, wherein said amplification regimen comprising at least two amplification reaction cycles, wherein each cycle comprises the steps of: 1) nucleic acid strand separation; 2) olignucleotide primer annealing; and 3) polymerase extension of annealed primers.

22. The method of claim 21 further comprising the steps, during said amplification regimen and after at least one of said reaction cycles, of removing an aliquot of said amplification reaction, separating nucleic acid molecules by size and/or by charge, and detecting the incorporation of a said distinguishable label, wherein said detecting determines the identity of the nucleotide at said polymorphic site.

23. The method of claim 22, wherein said removing, separating and detecting are performed after each cycle in said regimen.

24. The method of claim 18, wherein steps I-VI are performed in a modular apparatus comprising a thermal cycle, a sampling device, a capillary electrophoresis device and a fluorescent detector.

25. The method of claim 18, wherein said tag sequences each comprise 15 to 40 nucleotides.

26. The method of claim 18, wherein said 3' region that hybridizes to a sequence at a known distance upstream of said known polymorphic site comprises 10-30 nucleotides.

27. The method of claim 18 wherein said region that hybridizes 3' of and adjacent to said polymorphic site comprises 10-30 nucleotides.

28. The method of claim 18 wherein said set of downstream amplification primers consists of: a subset that comprises a tag sequence that specifically corresponds to the presence of A at the polymorphic site; a subset that comprises a tag sequence that specifically corresponds to the presence of C at the polymorphic site; a subset that comprises a tag sequence that specifically corresponds to the presence of G at the polymorphic site; and a subset that comprises a tag sequence that specifically corresponds to the presence of T at the polymorphic site.

29. A kit for the determination of the nucleotide present at a polymorphic site present on a nucleic acid sample, said kit comprising a set of upstream primers comprising:
(a) a first primer comprising a 5'-tag sequence and 3' sequence sufficient to specifically hybridize at a known distance upstream of a known polymorphic site; and
(b) a set of 4 downstream second primers, comprising in 5' to 3' order:
(i) a sequence tag selected from the group consisting of a tag specifically corresponding to G as the 3'-terminal nucleotide of said primer; a tag specifically corresponding to A as the 3'-terminal nucleotide of said primer; a tag specifically corresponding to T as the 3'-terminal nucleotide of said primer; and a tag specifically corresponding to C as the 3'-terminal nucleotide of said primer;
(ii) a region that specifically hybridizes to a sequence adjacent to and 3' or a polymorphic site in said group of polymorphic sites, wherein said set of downstream amplification primers comprises a subset of primers comprising a region that specifically hybridizes adjacent to said polymorphic site for each polymorphic site in said group of polymorphic sites; and
(iii) a 3' terminal nucleotide selected from G, A, T or C, wherein said terminal nucleotide specifically corresponds to the sequence tag described in (i) on that downstream amplification primer, and wherein when said downstream amplification primer is hybridized to said sequence adjacent to and 3' of a polymorphic site, said 3' terminal nucleotide is opposite said polymorphic site.

30. The kit of claim 29 further comprising a set of 5 primers lacking sequence specific for a gene in the genome of the organism being examined for polymophisms, said primers comprising a primer comprising the tag sequence of said first primer and a set of four distinguishably labelled primers comprising the tag sequences of said set of four downstream second primers.

## Patentansprüche

1. Verfahren, um für eine vorgegebene Nukleinsäureprobe die Identitäten der Nukleotide an einen Satz abzufragender, bekannter polymorpher Stellen zu bestimmen, wobei das Verfahren folgendes umfasst:
(a) dass man eine Population von Primerverlängerungsprodukten, die von einer Nukieinsäuneprobe erzeugt wurden, einem Vervielfältigungssystam unterwirft, wobei jedes Primerverlängerungsprodukt ein Mitglied aus einem Satz von Markierungssequenzen umfasst, wobei die Marklerungssequenz spezifisch dem Vorhandensein eines spezifischen Nukleotids an einer bekannten polymorphen Stelle entspricht, wobei das Vervielfältigungssystem unter Verwendung eines stromaufwärts liegenden Vervielfältigungsprimers für jede Sequenz, die eine abzufragende, bekannte polymorphe Stelle umfasst, und eines Satzes von unterscheidbar markierten, stromabwärts liegenden Vervielfältigungsprimem angewendet wird, wobei jedes Mitglied des Satzes von stromabwärts liegenden Vervielfältigungsprimem eine genannte Markierungssequenz enthält, die ein Mitglied der Population von Primerverlängerungsprodukten und eine unterscheidbare Markierung, die spezifisch dem Vorhandensein eines spezifischen Nukleotids an der polymorphen Stelle entspricht, umfasst und wobei die stromaufwärts liegenden Vervielfältigungsprimer so ausgewählt sind, dass jede polymorphe Stelle des Satzes abzufragender, bekannter polymorpher Stellen einem Vervielfältigungsprodukt mit unterscheidbarer Größte entspricht;
(b) dass man die Nukleinsäuremoleküle, die unter dem Vervielfältigungssystem erzeugt wurden, nach Größe und/oder nach Ladung trennt;
(c) dass man die Aufnahme eines unterscheidbar markierten, stromabwärts liegenden Vervlelfältigungsprimers in Vervielfältigungsprodukte von unterscheidbarer Größe aus Stufe (a) feststellt, um dabei die Identität des Nukleotide an jeder der polymorphen Stellen zu bestimmen.

2. Verfahren nach Anspruch 1, wobei jedes Prirnerveriängerungsprodukt ein Mitglied aus einem Satz von ersten Marklerungssequenzen oder dessen Komplementäres und eine zweite Markierungssequenz oder deren Komplementäres umfasst, wobei das Vorhandensein der zweiten Markierungssequenz oder ihres Komplementäre spezifisch dem Vorhandensein eines spezifischen Nukleotids an einer bekannten polymorphen Stelle entspricht, wobei für jede polymorphe Stelle in dem Satz von polymorphen Stellen die erste Markierungssequenz in einem unterscheidbaren Abstand 5' von der polymorphen Stelle relativ zu dem Abstand der ersten Markierungssequenz von einer polymorphen Stelle auf Molekülen in der Probe, die andere polymorphe Stellen enthalten, angeordnet ist, wobei das Vervielfältigungssystem unter Verwenden des stromaufwärts liegenden Vervielfältigungsprimers, der die erste Markierungssequenz umfasst, und eines Satzes von unterscheidbar markierten, stromabwärts gelegenen Vervielfältigungsprimem durchgeführt wird, wobei jedes Mitglied von dem Satz von stromabwärts gelegenen Vervielfältigungsprimem eine genannte zweite Markierungssequenz enthält, die von einem Mitglied der Population von Primerverlängerungsprodukten und einer unterscheidbaren Markierung, die spezifisch dem Vorhandensein eines spezifischen Nukleotids and er polymorphen Stelle entspricht, umfasst ist, und wobei die stromaufwärts gelegenen Vervielfältigungsprimer so ausgewählt sind, dass jede polymorphe Stelle des Satzes von abzufragenden, bekannten polymorphen Stellen einem Vervlelfältigungsprodukt mit unterscheidbarer Größe entspricht.

3. Verfahren nach Anspruch 1, wobei die unterscheidbare Markierung eine Fluoreszenzmarkierung ist.

4. Verfahren nach Anspruch 1, wobei das Trennen Kapillarelektrophorese umfasst.

5. Verfahren nach Anspruch 1, wobei das Vervielfältigungssystem wenigstens zwei Vervielfältigungsreaktionszyklen umfasst, von denen jeder Zyklus die folgenden Stufen beinhaltet: (1) Nukleinsäurestrangtrennung: (2) Oligonukleotidprimer-Annealen und (3) Polymerase-Verlängerung von annealten Primem.

6. Verfahren nach Anspruch 5, welches weiterhin die Stufen umfasst, bei denen man unter dem Vervielfältigungssystem und nach wenigstens einem der Reaktionszyklen einen aliquoten Teil der Vervielfältigungsreaktion abnimmt, Nukleinsäuremoleküle nach Größe und/oder nach Ladung trennt und die Aufnahme einer genannten unterscheidbaren Markierung feststellt, wobei durch das Feststellen die Identität des Nukleotids an der polymorphen Stelle bestimmt wird.

7. Verfahren nach Anspruch 6, wobei das Abnehmen, Trennen und Feststellen nach jedem Zyklus in dem System durchgeführt wird.

8. Verfahren nach Anspruch 6, wobei das Trennen Kapillarelektrophorese umfasst

9. Verfahren nach Anspruch 1, wobei die Stufen (a) und (b) in einer modularen Vorrichtung durchgeführt werden, die ein Temperaturwechseigerät, eine Probennahmevorrichtung, eine Kapillarelektrophoresevorrichtung und einen Ftuoreszenzdetektor umfasst.

10. Verfahren nach Anspruch 1, wobei die Markierungssequenz 15 bis 40 Nukleotide umfasst.

11. Verfahren nach Anspruch 1, wobei der Satz von unterscheidbar markierten, stromabwärts liegenden Vervielfältigungsprimem aus folgendem besteht: einer Teilmenge, die eine Markiarungssequenz aufweist, die spezifisch dem Vorhandensein von A an der polymorphen Stelle entspricht, einer Teilmenge, die eine Markierungssequenz aufweist, die spezifisch dem Vorhandenseln von C an der polymorphen Stelle entspricht, einer Teilmenge, die eine Markierungssequenz aufweist, die spezifisch dem Vorhandensein von G an der polymorphen Stelle entspricht, und einer Teilmenge, die eine Marklerungssequenz aufweist, die spezifisch dem Vorhandensein von T an der polymorphen Stelle entspricht.

12. Verfahren nach Anspruch 1, wobei der Satz von unterscheidbar markierten, stromabwärts gelegenen Vervieifäitigungsprimem aus einem Paar von Ollgonukleotiden besteht, von denen eines eine Marklerungssequenz umfasst, die spezifisch einer ersten Allele der polymorphen Stelle entspricht, und eines eine Markierungssequenz umfasst, die spezifisch einer zweiten Allele der polymorphen Stelle entspricht.

13. Verfahren nach Anspruch 1, welches vor der Stufe (a) weiterhin die Stufe umfasst, bei der man Primer, die, wenn die Population von Primanrerlängerungsprodukten hergestellt wurde, nicht aufgenommen wurden, entfernt.

14. Verfahren nach Anspruch 13, wobei die Stufe des Entfernens den Abbau der Primer umfasst, die, wenn die Population von Primerverlängerungsprodukten hergestellt wurde, nicht aufgenommen wurden.

15. Verfahren nach Anspruch 14, wobei der Abbau unter Verwendung einer hitzelabilen Exonuklease durchgeführt wird.

16. Verfahren nach Anspruch 15, wobei die hitzelabile Exonuklease aus der Gruppe ausgewählt ist, bestehend aus Exonuklease I und Exonuklease VII.

17. Verfahren nach Anspruch 16, wobei die hitzelablle Exonuklease thermisch inaktiviert wird, bevor man mit Stufe (a) fortfährt.

18. Verfahren nach Anspruch 1, wobei die Population von Primerverlängerungsprodukten nach einem Verfahren erzeugt wird, weiches folgendes umfasst:
I) Bereitstellen einer Nukleinsäureprobe, welche die Gruppe von polymorphen Stellen umfasst,
II) Trennen der Stränge dieser Nukleinsäureprobe und erneutes Annealen in der Gegenwart von
a) einem Satz von ersten Oligonukleotidprimern, von denen jeder einen 3'-Bereich aufweist, der an eine Sequenz in einem bekannten Abstand stromaufwärts von einer bekannten polymorphen Stelle hybridisiert, wobei jedes Mitglied des Satzes von ersten Oligonukleotidprimem eine 5' zu dem 3'-Bereich angeordnete Markierung einer gemeinsamen Sequenz aufweist und jedes Mitglied des Satzes von ersten Oligonukleotidprimern so ausgewählt ist, dass für jede polymorphe Stelle in der Gruppe von bekannten polymorphen Stellen ein Vervielfältigungsprodukt mit unterscheidbarer Größe erzeugt wird, und
b) einem Satz von stromabwärts liegenden Vervielfältigungsprimem, die in der Richtung von 5' nach 3' folgendes umfassen:
i) eine Sequenzmarkierung, ausgewählt aus der Gruppe, bestehend aus einer Markierung, die spezifisch G als dem 3'-terminalen Nukleotid des Primers entspricht, einer Markierung, die spezifisch A als dem 3'-terminalen Nukleotid des Primers entspricht, und einer Markierung, die spezifisch T als dem 3'-terminalen Nukleotid des Primers entspricht, und einer Markierung, die spezifisch C als dem 3'terminalen Nukleotid des Primers entspricht,
ii) einen Bereich, der spezifisch an eine Sequenz in der Nähe zu und 3' von einer polymorphen Stelle in der Gruppe von polymorphen Stellen hybridisiert, wobei der Satz von stromabwärts liegenden Vervielfältigungsprimem eine Untergruppe von Primem umfasst, die einen Bereich aufweisen, der für jede polymorphe Stelle in der Gruppe von polymorphen Stellen spezifisch in der Nähe der polymorphen Stelle hybridisiert, und
iii) ein 3'-terminales Nukleotid, ausgewählt unter G, A, T oder C, wobei das terminale Nukleotid spezifisch der in (i) beschriebenen Sequenzmarkierung auf dem stromabwärts liegenden Vervielfältigungsprimer entspricht und wobei das 3'-terminale Nukleotid gegenüber einer polymorphen Stelle liegt, wenn der stromabwärts liegende Vervielfältigungsprimer an die Sequenz in der Nähe zu und 3' von einer polymorphen Stelle hybridisiert,
III) in Kontakt bringen der annealten Oligonukleotide, die aus Stufe (II) resultieren, mit einer Nukleinsäurepolymerase unter Bedingungen, weiche die Verlängerung eines annealten Oligonukleotids erlauben, so dass Veriängerungsprodukte erzeugt werden, wobei das Primerverlängenangsprodukt von dem ersten Oligonukleotidprimer, wenn er von seinem Komplementären getrennt ist, als eine Matrize für die Synthese des Verlängerungsproduktes als ein Mitglied des Satzes von zweiten Oligonukleotidprimem dienen kann und umgekehrt,
IV) zweimaliges Wiederholen der Stufen (II) und (III) der Strangtrennung und des in Kontakt bringens, so dass ein Reaktionsgemisch mit einer Population von Nukleinsäuremolekülen erzeugt wird, das sowohl eine zu dem ersten Oligonukleotid identische oder komplementäre Sequenz als auch eine zu einem Mitglied des Satzes von stromabwärts liegenden Vervielfältigungsprimem identische oder komplementäre Sequenz enthält,
V) in Kontakt bringen der in Stufe (IV) erzeugten Population mit einer hitzelabilen Exonuklease unter Bedingungen, welche den Abbau von nicht annealten Oligonukleotidprimem erlauben, so dass nicht annealte Primer abgebaut werden,
VI) thermisches Inaktivieren der hitzelabilen Exonuklease,
und wobei die Stufe, bei der man eine Population von Primerverlängerungsprodukten einem Vervlelfältigungssystem unterwirft, unter Verwendung eines stromaufwärts liegenden Primers durchgeführt wird, weicher die gemeinsame Sequenzmarkierung umfasst, die der erste Oligonukleatidprimer aufweist,
und einen Satz von stromabwärts liegenden Vervielfältigungsprimern, wobei jedes Mitglied des Satzes von stromabwärts liegenden Vervielfältigungsprimem eine Markierung umfasst, die in einem Mitglied des Satzes von zweiten Oligonukleotidpimern enthalten ist, und eine unterscheidbare Markierung.

19. Verfahren nach Anspruch 18, wobei die unterscheidbare Markierung eine Fiuoreszenzmarkierung ist.

20. Verfahren nach Anspruch 18, wobei die Trennung Kapillarelektrophorese umfasst.

21. Verfahren nach Anspruch 18, wobei das Vervielfältigungssystem wenigstens zwei Vervielfältigungsreaktionszyklen umfasst, bei denen jeder Zyklus die folgenden Stufen aufweist: 1) Nukleinsäurestrangtrennung, 2) Oligonukleotidprimer-Anneafen und 3) Polymeraseverlängerung von annealten Primem.

22. Verfahren nach Anspruch 21, welches weiterhin die Stufen umfasst, bei denen man unter dem Vervielfältigungssystem und nach wenigstens einem der Reaktionszyklen einen aliquoten Teil der Vervielfältigungsreaktion abnimmt, Nukleinsäuremoleküle nach Größe und/oder nach Ladung trennt und die Aufnahme einer unterscheidbaren Markierung feststellt, wobei das Feststellen die Identität des Nukleotids an der polymorphen Stelle bestimmt.

23. Verfahren nach Anspruch 22, wobei das Abnehmen, Trennen und Feststellen nach jedem Zyklus in dem System durchgeführt wird.

24. Verfahren nach Anspruch 18, wobei die Stufen I-VI in einer modularen Vorrichtung durchgeführt werden, die eine Temperaturwechselapparatur, eine Probennahmevorrichtung, eine Kapillarelektrophoresevorrichtung und einen Fluoreszenzdetektor umfasst.

25. Verfahren nach Anspruch 18, wobei die Markierungssoquenzen jeweils 15 bis 40 Nukleotide umfassen.

26. Verfahren nach Anspruch 18, wobei der 3'-Bereich, der an eine Sequenz in einem bekannten Abstand stromaufwärts zu der bekannten polymorphen Stelle hybridisiert, 10 bis 30 Nukleotide umfasst.

27. Verfahren nach Anspruch 18, wobei der Bereich, der 3' von und in der Nähe zu der polymorphen Stelle hybridisiert, 10 bis 30 Nukleotide umfasst.

28. Verfahren nach Anspruch 18, wobei der Satz von stromabwärts liegenden Vervielfältigungsprimem aus folgendem besteht: einer Teilmenge, die eine Markierungssequenz aufweist, die spezifisch dem Vorhandensein von A an der polymorphen Stelle entspricht, einer Teilmenge, die eine Markierungssequenz aufweist, die spezifisch dem Vorhandensein von C an der polymorphen Stelle entspricht, einer Teilmenge, die eine Marklerungssequenz aufweist, die spezifisch dem Vorhandensein von G an der polymorphen Stelle entspricht, und einer Teilmenge, die eine Markierungssequenz aufweist, die spezifisch dem Vorhandensein von T an der polymorphen Stelle entspricht.

29. Kit für die Bestimmung des Nukleotids, das an einer auf einer Nukleinsäureprobe vorhandenen polymorphen Stelle vorhanden ist, wobei der Kit einen Satz von stromaufwärts liegenden Primem umfasst, die folgendes aufweisen:
(a) einen ersten Primer, der eine 5'-Markierungssequenz und eine 3-sequenz aufweist, die ausreichend sind, um spezifisch in einem bekannten Abstand stromaufwärts von einer bekannten polymorphen Stelle zu hybridisieren, und
(b) einen Satz von 4 stromabwärts liegenden zweiten Primem, die in der Richtung von 5' nach 3' folgende umfassen:
(i) eine Sequenzmarkierung, ausgewählt aus der Gruppe, bestehend aus einer Markierung, die spezifisch G als dem 3'-terminalen Nukleotid des Primers entspricht, einer Markierung, die spezifisch A als dem 3'-terminalen Nukleotid des Primers entspricht, und einer Markierung, die spezifisch T als dem 3'-terminalen Nukleotid des Primers entspricht, und einer Markierung, die spezifisch C als dem 3'terminale Nukleotid des Primers entspricht,
(ii) einen Bereich, der spezifisch an eine Sequenz in der Nähe zu und 3' von einer polymorphen Stelle in der Gruppe von polymorphen Stellen hybridisiert, wobei der Satz von stromabwärts liegenden Vervielfältigungsprimem eine Untergruppe von Primem umfasst, die einen Bereich aufweisen, der für jede polymorphe Stelle in der Gruppe von polymorphen Stellen spezifisch in der Nähe der polymorphen Stelle hybridosiert, und
(iii) ein 3'-terminals Nukleotid, ausgewählt unter G, A, T oder C, wobei das terminale Nukleotid spezifisch der in (i) beschriebenen Sequenz markierung auf dem stromabwärts liegenden Vervielfältigungsprimer entspricht und wobei das 3'-terminale Nukleotid gegenüber einer polymorphen Stelle liegt, wenn der stromabwärts liegende Vervielfältigungsprimer an die Sequenz in der Nähe zu und 3' von einer polymorphen Stelle hybridisiert.

30. Kit nach Anspruch 29, welcher weiterhin einen Satz von 5 Primern aufweist, denen eine Sequenz fehlt, die für ein Gen in dem Genom des hinsichtlich Polymorphismen untersuchten Organismus spezifisch ist, wobei die Primer einen Primer umfassen, der die Markierungssequenz von dem ersten Primer aufweist, und einen Satz von vier unterscheidbar markierten Primern, welche die Markierungssequenzen von dem Satz von vier stromabwärts liegenden zweiten Primern ausweisen.

## Revendications

1. Procédé de détermination, pour un échantillon d'acide nucléique donné, des identités des nucléotides au niveau d'un ensemble de sites polymorphiques connus à interroger, ledit procédé comprenant :
(a) la soumission à un régime d'amplification, d'une population de produits d'extension d'amorces générés à partir d'un échantillon d'acide nucléique, chaque produit d'extension d'amorce comprenant un membre d'un ensemble de séquences marqueurs, laquelle séquence marqueur correspond à la présence d'un nucléotide spécifique au niveau d'un site polymorphique connu, où ledit régime d'amplification est réalisé en utilisant une amorce d'amplification en amont pour chaque séquence comprenant un site polymorphique connu à interroger, et un ensemble d'amorces d'amplification en aval marquées de manière distinctive, chaque membre dudit ensemble d'amorphes d'amplification en aval comprenant une dite séquence marqueur composée d'un membre de ladite population de produits d'extension d'amorces et d'un marqueur pouvant être distingué qui correspond spécifiquement à la présence d'un nucléotide spécifique au niveau dudit site polymorphique, et où lesdites amorces d'amplification en amont sont choisies de telle manière que chaque site polymorphique dudit ensemble de sites polymorphiques connus à interroger correspond à un produit d'amplification de taille distincte ;
(b) la séparation des molécules d'acide nucléique fabriquées durant ledit régime d'amplification par taille et/ou par charge ;
(c) la détection de l'incorporation d'une amorce d'amplification en aval marquée de manière distinctive dans les produits d'amplification de taille distincte de l'étape (a), pour déterminer de cette manière l'identité du nucléotide au niveau de chaque dit site polymorphique.

2. Procédé selon la revendication 1, dans lequel chaque produit d'extension d'amorce comprend un membre d'un ensemble d'une première séquence marqueur ou son complémentaire et d'une seconde séquence marqueur ou son complémentaire, dont la présence de la seconde séquence marqueur ou de son complémentaire correspond spécifiquement à la présence d'un nucléotide spécifique au niveau d'un site polymorphique connu, où pour chaque site polymorphique dans ledit ensemble de sites polymorphiques, ladite première séquence marqueur est localisée à une distance distincte en 5' dudit site polymorphique, par rapport à la distance de ladite premières séquence marqueur à partir d'un site polymorphique, sur des molécules dans ledit échantillon contenant d'autres sites polymorphiques, où ledit régime d'amplification est réalisé en utilisant une amorce d'amplification en amont comprenant ladite première séquence marqueur, et un ensemble d'amorces d'amplification en aval marquées de manière distinctive, chaque membre dudit ensemble d'amorces d'amplification en aval comprenant une dite seconde séquence marqueur composée d'un membre de ladite population de produits d'extension d'amorces et d'un marqueur pouvant être distingué qui correspond spécifiquement à la présence d'un nucléotide spécifique au niveau dudit site polymorphique, et où lesdites amorces d'amplification en amont sont choisies de telle manière que chaque site polymorphique dudit ensemble de sites polymorphiques connus à interroger correspond à un produit d'amplification de taille distincte.

3. Procédé selon la revendication 1, dans lequel ledit marqueur pouvant être distingué est un marqueur fluorescent.

4. Procédé selon la revendication 1, dans lequel ladite séparation comprend l'électrophorèse capillaire.

5. Procédé selon la revendication 1, dans lequel ledit régime d'amplification comprend au moins deux cycles de réaction d'amplification, où chaque cycle comprend les étapes suivantes : (1) la séparation des brins d'acide nucléique ; (2) l'hybridation des amorces oligonucléotidiques ; et (3) l'extension par la polymérase des amorces hybridées.

6. Procédé selon la revendication 5, comprenant en outre les étapes, durant ledit régime d'amplification et après au moins l'un desdits cycles réactionnels, de prélèvement d'un aliquot de ladite réaction d'amplification, de séparation des molécules d'acide nucléique par taille et/ou par charge, et de détection de l'incorporation d'un dit marqueur pouvant être distingué, où ladite détection détermine l'identité du nucléotide au niveau dudit site polymorphique.

7. Procédé selon la revendication 6, dans lequel lesdits prélèvement, séparation et détection sont réalisés après chaque cycle dans ledit régime.

8. Procédé selon la revendication 6, dans lequel ladite séparation comprend l'électrophorèse capillaire.

9. Procédé selon la revendication 1, dans lequel les étapes (a) et (b) sont réalisées dans un appareil modulaire comprenant un thermocycleur, un dispositif d'échantillonnage, un dispositif d'électrophorèse capillaire et un détecteur de fluorescence.

10. Procédé selon la revendication 1, dans lequel ladite séquence marqueur comprend 15 à 40 nucléotides.

11. Procédé selon la revendication 1, dans lequel ledit ensemble d'amorces d'amplification en aval marquées de manière distinctive est constitué par : un sous-ensemble qui comprend une séquence marqueur qui correspond spécifiquement à la présence de A au niveau du site polymorphique ; un sous-ensemble qui comprend une séquence marqueur qui correspond spécifiquement à la présence de C au niveau du site polymorphique ; un sous-ensemble qui comprend une séquence marqueur qui correspond spécifiquement à la présence de G au niveau du site polymorphique ; et un sous-ensemble qui comprend une séquence marqueur qui correspond spécifiquement à la présence de T au niveau du site polymorphique.

12. Procédé selon la revendication 1, dans lequel ledit ensemble d'amorces d'amplification en aval marquées de manière distinctive est constitué d'une paire d'oligonucléotides, un comprenant une séquence marqueur qui correspond spécifiquement à un premier allèle du site polymorphique et un comprenant une séquence marqueur qui correspond spécifiquement à un second allèle du site polymorphique.

13. Procédé selon la revendication 1, comprenant en outre l'étape, avant l'étape (a), d'élimination des amorces non incorporées lorsque ladite population des produits d'extension d'amorces a été fabriquée.

14. Procédé selon la revendication 13, dans lequel ladite étape d'élimination comprend la dégradation desdites amorces non incorporées lorsque ladite population des produits d'extension d'amorces a été fabriquée.

15. Procédé selon la revendication 14, dans lequel ladite dégradation est réalisée en utilisant une exonucléase thermolabile.

16. Procédé selon la revendication 15, dans lequel ladite exonucléase thermolabile est choisie dans le groupe comprenant l'exonucléase I et l'exonucléase VII.

17. Procédé selon la revendication 16, dans lequel ladite exonucléase thermolabile est inactivée thermiquement avant de passer à l'étape (a).

18. Procédé selon la revendication 1, dans lequel ladite population de produits d'extension d'amorces est générée par un procédé comprenant :
I) la fourniture d'un échantillon d'acide nucléique comprenant ledit groupe de sites polymorphiques ;
II) la séparation des brins dudit échantillon d'acide nucléique et la réhybridation en présence de :
a) un ensemble de premières amorces oligonueléotidiques comprenant chacune une région en 3' qui s'hybride à une séquence à une distance connue en amont d'un site polymorphique connu, chaque membre dudit ensemble de premières amorces oligonucléotidiques comprenant un marqueur de séquence commun localisé en 5' de ladite région en 3', et chaque membre dudit ensemble de premières amorces oligonucléotidiques choisi de telle manière qu'un produit d'amplification de taille distincte est généré pour chaque site polymorphique dans ledit groupe de sites polymorphiques connus ; et
b) un ensemble d'amorces d'amplification en aval comprenant, dans l'ordre 5' à 3' :
i) un marqueur de séquence choisi dans le groupe comprenant un marqueur correspondant spécifiquement à G en tant que nucléotide terminal en 3' de ladite amorce ; un marqueur correspondant spécifiquement à A en tant que nucléotide terminal en 3' de ladite amorce et un marqueur correspondant spécifiquement à T en tant que nucléotide terminal en 3' de ladite amorce ; et un marqueur correspondant spécifiquement à C en tant que nucléotide terminal en 3' de ladite amorce ;
ii) une région qui s'hybride spécifiquement à une séquence adjacente à et en 3' d'un site polymorphique dans ledit groupe de sites polymorphiques, où ledit ensemble d'amorces d'amplification en aval comprend un sous-ensemble d'amorces comprenant une région qui s'hybride spécifiquement adjacente au dit site polymorphique pour chaque site polymorphique dans ledit groupe de sites polymorphiques ; et
iii) un nucléotide terminal en 3' choisi parmi G, A, T ou C, où ledit nucléotide terminal correspond spécifiquement au marqueur de séquence décrit en (i) sur cette amorce d'amplification en aval, et où lorsque ladite amorce d'amplification en aval est hybridée à ladite séquence adjacente à et en 3' d'un site polymorphique, ledit nucléotide terminal en 3' est opposé au dit site polymorphique ;
III) la mise en contact des oligonucléotides hybridés résultant de l'étape (II) avec une polymérase d'acide nucléique dans des conditions qui permettent l'extension d'un oligonucléotide hybridé de telle manière que des produits d'extension sont générés, où le produit d'extension d'amorce issu de la première amorce oligonucléotidique, lorsqu'il est séparé de son complémentaire, peut servir de matrice pour la synthèse du produit d'extension comme membre de l'ensemble des secondes amorces oligonucléotidiques, et vice versa ;
IV) la répétition des étapes (II) et (III) de séparation des brins et de mise en contact deux fois, de telle manière qu'un mélange réactionnel comprenant une population de molécules d'acide nucléique est généré, lequel comprend à la fois une séquence identique au ou complémentaire du dit premier oligonucléotide et une séquence identique à ou complémentaire d'un membre dudit ensemble d'amorces d'amplification en aval ;
V) la mise en contact de la population générée dans l'étape (IV) avec une exonucléase thermolabile dans des conditions permettant la dégradation des amorces oligonucléotidiques non hybridées, de telle manière que les amorces non hybridées sont dégradées ;
VI) l'inactivation thermique de ladite exonucléase thermolabile ;
et où ladite étape de soumission d'une population de produits d'extension d'amorces à un régime d'amplification est réalisée en utilisant une amorce d'amplification en amont comprenant le marqueur de séquence commun composé de ladite première amorce oligonucléotidique,
et un ensemble d'amorces d'amplification en aval, chaque membre dudit ensemble d'amorces d'amplification en aval comprenant un marqueur composé d'un membre dudit ensemble de secondes amorces oligonucléotidiques et un marqueur pouvant être distingué.

19. Procédé selon la revendication 18, dans lequel ledit marqueur pouvant être distingué est un marqueur fluorescent.

20. Procédé selon la revendication 18, dans lequel ladite séparation comprend l'électrophorèse capillaire.

21. Procédé selon la revendication 18, dans lequel ledit régime d'amplification comprenant au moins deux cycles de réaction d'amplification, où chaque cycle comprend les étapes suivantes : (1) la séparation des brins d'acide nucléique ; (2) l'hybridation des amorces oligonucléotidiques; et (3) l'extension par la polymérase des amorces hybridées.

22. Procédé selon la revendication 21, comprenant en outre les étapes, durant ledit régime d'amplification et après au moins l'un desdits cycles réactionnels, de prélèvement d'un aliquot de ladite réaction d'amplification, de séparation des molécules d'acide nucléique par taille et/ou par charge, et de détection de l'incorporation d'un dit marqueur pouvant être distingué, où ladite détection détermine l'identité du nucléotide au niveau dudit site polymorphique.

23. Procédé selon la revendication 22, dans lequel lesdits prélèvement, séparation et détection sont réalisés après chaque cycle dans ledit régime.

24. Procédé selon la revendication 18, dans lequel les étapes I à VI sont réalisées dans un appareil modulaire comprenant un thermocycleur, un dispositif d'échantillonnage, un dispositif d'électrophorèse capillaire et un détecteur de fluorescence.

25. Procédé selon la revendication 18, dans lequel lesdites séquences marqueurs comprennent chacune 15 à 40 nucléotides.

26. Procédé selon la revendication 18, dans lequel ladite région en 3' qui s'hybride à une séquence à une distance connue en amont dudit site polymorphique connu comprend 10 à 30 nucléotides.

27. Procédé selon la revendication 18, dans lequel ladite région qui s'hybride en 3' de et adjacente au dit site polymorphique comprend 10 à 30 nucléotides.

28. Procédé selon la revendication 18, dans lequel ledit ensemble d'amorces d'amplification en aval comprend : un sous-ensemble qui comprend une séquence marqueur qui correspond spécifiquement à la présence de A au niveau du site polymorphique ; un sous-ensemble qui comprend une séquence marqueur qui correspond spécifiquement à la présence de C au niveau du site polymorphique ; un sous-ensemble qui comprend une séquence marqueur qui correspond spécifiquement à la présence de G au niveau du site polymorphique ; et un sous-ensemble qui comprend une séquence marqueur qui correspond spécifiquement à la présence de T au niveau du site polymorphique.

29. Kit pour la détermination du nucléotide présent au niveau d'un site polymorphique présent dans un échantillon d'acide nucléique, ledit kit comprenant un ensemble d'amorces en amont comprenant :
(a) une première amorce comprenant une séquence marqueur en 5' et une séquence en 3' suffisante pour s'hybrider spécifiquement à une distance connue en amont d'un site polymorphique connu ; et
(b) un ensemble de 4 secondes amorces en aval, comprenant dans l'ordre 5' à 3' :
i) un marqueur de séquence choisi dans le groupe comprenant un marqueur correspondant spécifiquement à G en tant que nucléotide terminal en 3' de ladite amorce ; un marqueur correspondant spécifiquement à A en tant que nucléotide terminal en 3' de ladite amorce et un marqueur correspondant spécifiquement à T en tant que nucléotide terminal en 3' de ladite amorce ; et un marqueur correspondant spécifiquement à C en tant que nucléotide terminal en 3' de ladite amorce ;
ii) une région qui s'hybride spécifiquement à une séquence adjacente à et en 3' d'un site polymorphique dans ledit groupe de sites polymorphiques, où ledit ensemble d'amorces d'amplification en aval comprend un sous-ensemble d'amorces comprenant une région qui s'hybride spécifiquement adjacente au dit site polymorphique pour chaque site polymorphique dans ledit groupe de sites polymorphiques ; et
iii) un nucléotide terminal en 3' choisi parmi G, A, T ou C, où ledit nucléotide terminal correspond spécifiquement au marqueur de séquence décrit en (i) sur cette amorce d'amplification en aval, et où lorsque ladite amorce d'amplification en aval est hybridée à ladite séquence adjacente à et en 3' d'un site polymorphique, ledit nucléotide terminal en 3' est opposé au dit site polymorphique.

30. Kit selon la revendication 29, comprenant en outre un ensemble de 5 amorces manquant de séquence spécifique d'un gène dans le génome de l'organisme examiné pour des polymorphismes, lesdites amorces comprenant une amorce comprenant la séquence marqueur de ladite première amorce et un ensemble de quatre amorces marquées de manière distinctive comprenant les séquences marqueurs dudit ensemble de quatre secondes amorces en aval.
